# EUROPEAN PATENT APPLICATION

(11) **EP 4 726 293 A1**
(43) Date of publication of application: **15.04.2026**
(21) Application number: 24818757.7
(22) Date of filing: 07.06.2024
(51) Int. Cl.: F25D 11/02

(54) **LOW-TEMPERATURE BIOLOGICAL SAMPLE PRESERVATION CABINET**

(30) Priority: 08.06.2023 CN 202310676270; 08.06.2023 CN 202321459081 U; 08.06.2023 CN 202310681727; 08.06.2023 CN 202321465897 U; 08.06.2023 CN 202321454126 U; 08.06.2023 CN 202321459104 U; 15.09.2023 CN 202311190877; 15.09.2023 CN 202322507886 U; 13.11.2023 CN 202323061781 U
(71) Applicant: Genepoint Technologies (Shanghai) Co., Ltd., Shanghai 201206 (CN)
(72) Inventor: DING, Fubin, Shanghai 201206 (CN); CHEN, Zhehua, Shanghai 201206 (CN); LI, Huang, Shanghai 201206 (CN); ZOU, Jimmy, Shanghai 201206 (CN); PENG, Jing, Shanghai 201206 (CN); JIANG, Jakalen, Shanghai 201206 (CN); LUO, Youzhi, Shanghai 201206 (CN)
(74) Representative: Wang, Bo
(86) International application number: PCT/CN2024/097929
(87) International publication number: WO 2024/251226

(57) **Abstract**

The present disclosure relates to a low-temperature preservation cabinet for a biological sample, including: a cabinet, the cabinet being provided with a storage region and an equipment region. The storage region is configured to store the biological sample. The temperature isolation region is provided between the storage region and the equipment region. A temperature of the storage region is lower than a temperature of the equipment region.

## Description

### CROSS-REFERENCE TO RELATED APPLICATIONS

This application is a Continuation of International Application No. PCT/CN 2024/097929, filed on June 7, 2024, which claims Chinese Application NO. 202310676270.3, filed on June 08, 2023, Chinese Application NO. 202321459081.2, filed on June 08, 2023, Chinese Application NO. 202310681727.X, filed on June 08, 2023, Chinese Application NO. 202321465897.6, filed on June 08, 2023, Chinese Application NO. 202321454126.7, filed on June 08, 2023, Chinese Application NO. 202321459104.X, filed on June 08, 2023, Chinese application 202311190877.7, filed on September 15, 2023, Chinese Application NO. 202322507886.6, filed on September 15, 2023, and Chinese Application NO. 202323061781.9, filed on November 13, 2023, the entire contents of each of which are hereby incorporated by reference.

### TECHNICAL FIELD

The present disclosure relates to the technical field of biological sample preservation, and in particular, to low-temperature preservation cabinets for biological samples.

### BACKGROUND

A preservation cabinet for a biological sample is a professional refrigerator that utilizes a low-temperature environment (e.g., it can be a low-temperature environment of minus 50° to minus 90°, or lower or higher temperatures, depending on the type of biological sample, etc.) for the storage of clinical biological samples, etc. With the rapid development of the biomedical industry, large biobanks have emerged, and the volume of stored biological samples has been increasing. Typically, biological samples such as tissues and cells from clinical settings or laboratories are stored in a low-temperature preservation cabinet for a biological sample.

### SUMMARY

One or more embodiments of the present disclosure provide a low-temperature preservation cabinet for a biological sample. The cabinet may include a cabinet body and a temperature isolation region. The cabinet body may be provided with a storage region and an equipment region, and the storage region may be configured to store the biological sample. The temperature isolation region may be provided between the storage region and the equipment region, and a temperature of the storage region may be lower than a temperature of the equipment region.

In some embodiments, the cabinet may further include a sampling mechanism and a refrigeration mechanism. The storage region may be provided with a biological sample rack and a sampling travel channel. The biological sample rack may be configured to place the biological sample, and a gap or a temperature isolation device may be provided between the storage region and the equipment region. The sampling mechanism may include a manipulator mechanism and a drive system. The drive system may be mounted in the equipment region, and the drive system may be configured to drive the manipulator mechanism to move in the sampling travel channel. The manipulator mechanism may be configured to pick up and place the biological sample. The refrigeration mechanism may be configured to control the temperature of the storage region to be within a temperature range not higher than -70 °C and to control the temperature of the equipment region to be within a temperature range not lower than -50 °C.

In some embodiments, the equipment region may be provided above, below, or at a side surface of the storage region.

In some embodiments, a gap may be provided between the storage region and the equipment region, and the gap takes any value within a range of 80 mm-200 mm.

In some embodiments, the equipment region is provided above the storage region, and the temperature isolation device includes a temperature isolation foam.

In some embodiments, a temperature isolation device may be provided between the storage region and the equipment region. The temperature isolation device may be provided with a passageway connecting the storage region and the equipment region. One end of the manipulator mechanism may pass through the passageway and may be connected to the drive system.

In some embodiments, the cabinet body may be provided with a maintenance port, the sampling mechanism may include a mounting frame, the drive system may be mounted on the mounting frame, and the mounting frame, the drive system, and the manipulator mechanism may be configured to slide and pull out of the maintenance port simultaneously.

In some embodiments, support strips may be symmetrically provided on opposite side walls in the cabinet body, the support strips may extend towards the maintenance port, the mounting frame may be a rectangular frame, and two sides of the mounting frame v placed on the support strips.

In some embodiments, one end of each of the support strips back away from the maintenance port may be provided with a bump perpendicular to the support strip, the bump may be provided with a positioning groove, and the mounting frame may be provided with a positioning pin corresponding to the positioning groove.

In some embodiments, the drive system may include a vertical drive motor, a transverse drive motor, a longitudinal drive motor, and a three-axis transmission mechanism mounted on the mounting frame. The three-axis transmission mechanism may be connected with the manipulator mechanism, the vertical drive motor, the transverse drive motor, and the longitudinal drive motor respectively. The vertical drive motor may drive the manipulator mechanism to move along a vertical direction through the three-axis transmission mechanism. The transverse drive motor may drive the manipulator mechanism to move along a horizontal and transverse direction through the three-axis transmission mechanism. The longitudinal drive motor may drive the manipulator mechanism to move along a horizontal and longitudinal direction through the three-axis transmission mechanism.

In some embodiments, the biological sample rack may include a first rack body and a second rack body. The first rack body may be symmetrically provided on two opposite inner walls of the cabinet body, the second rack body may be provided in the first rack body and parallel to the first rack body, and the second rack body may be configured as a back-to-back bidirectional rack body.

In some embodiments, the manipulator mechanism may include a pick-up arm configured to pick up and place the biological sample and a steering mechanism. The steering mechanism may include a rotating rack, a manipulator motor, and an offset guiding mechanism. The pick-up arm may be provided on the rotating rack, and the offset guiding mechanism may be connected with the rotating rack. The manipulator motor may be connected respectively to the rotating rack and the offset guiding mechanism for driving the rotating rack to rotate and for driving the offset guiding mechanism to move to drive the rotating rack to slide in a center axis direction during rotation.

In some embodiments, the cabinet body may include an accommodating cavity, and the accommodating cavity may include the storage region, the equipment region, and the temperature isolation region. The storage region may be further configured to place a manipulator mechanism, the equipment region may be configured to place a driving device, the driving device may be configured to drive the manipulator mechanism to pick up and place the biological sample, and the temperature isolation region may be provided with a temperature isolation device. The low-temperature preservation cabinet may further include an evaporator. The evaporator may be provided in a region of the cabinet body corresponding to the storage region, and in the temperature isolation region, so as to cooperate with the temperature isolation device to cause a temperature of the storage region to be lower than a temperature of the equipment region.

In some embodiments, the temperature isolation device includes a divider member. The divider member may be configured to connect a portion of the cabinet body forming the storage region and a portion of the cabinet body forming the equipment region, to minimize heat conduction between the portion of the cabinet body forming the storage region and the portion of the cabinet body forming the equipment region the portions.

In some embodiments, the divider member may at least include a first divider portion extending in a first direction, a second divider portion extending in a second direction different from the first direction, and a connection portion configured to connect the first divider portion and the second divider portion.

In some embodiments, the connecting portion may be provided with one of an insertion projection and an insertion slot, and the first divider portion and the second divider portion may be provided with another of the insertion projection and the insertion slot.

In some embodiments, the cabinet may further include at least one mounting box. Each of the at least one mounting box may be mounted in the temperature isolation region and may be provided with at least a portion of the evaporator, a side of the mounting box near the storage region may be provided with an opening, and a side of the mounting box close to the equipment region may be of a closed structure.

In some embodiments, the at least a portion of the evaporator provided within the mounting box may be provided on the side of the mounting box close to the storage region. The temperature isolation device may include a temperature isolation member provided on the side of the mounting box close to the equipment region to reduce a cooling capacity generated by the at least a portion of the evaporator within the mounting box to be transferred to the equipment region.

In some embodiments, the at least one mounting box may include a plurality of mounting boxes spaced apart.

In some embodiments, the cabinet may further include a compressor provided on a first side of the cabinet body and located outside the accommodating cavity. The compressor may be configured to form a refrigeration system together with the evaporator. The plurality of mounting boxes may include a first mounting box provided in a region of the temperature isolation region corresponding to the first side; and a second mounting box provided in a region of the temperature isolation region corresponding to a second side opposite the first side. The evaporator may be configured to be connected to the compressor after first entering the second mounting box and then the first mounting box.

In some embodiments, the plurality of mounting boxes may further include a third mounting box. The third mounting box may be provided between the first mounting box and the second mounting box, and the third mounting box may be connected with the first mounting box by a connection member, so as to allow the evaporator to first enter the second mounting box, then enter the first mounting box, then enter the third mounting box through the connection member, then enter the first mounting box through the connection member and then to be connected with the compressor, and the third mounting box may be spaced apart from the second mounting box.

In some embodiments, the equipment region may be located above the storage region.

In some embodiments, the cabinet body may be provided with a maintenance port. The storage region may be provided with a biological sample rack and a sampling travel channel, and the biological sample rack may be configured to place the biological sample. The equipment region may be provided with a mounting frame, and a drive system fixedly connected to the mounting frame. The drive system may be connected to a manipulator mechanism and configured to drive the manipulator mechanism to move in the sampling travel channel. The manipulator mechanism may be configured to pick up and place the biological sample. The mounting frame may be configured to slide and pull out of the maintenance port.

In some embodiments, support strips may be symmetrically provided on opposite side walls in the cabinet body. The support strips may extend towards the maintenance port, the mounting frame may be a rectangular frame, and two sides of the mounting frame may be placed on the support strips.

In some embodiments, one end of each of the support strips back away from the maintenance port may be provided with a bump perpendicular to the support strip, and the bump may be provided with a positioning groove. The mounting frame may be provided with a positioning pin corresponding to the positioning groove.

In some embodiments, the equipment region may be provided above, below, or on a side surface of the storage region.

In some embodiments, the drive system may include a vertical drive assembly. The vertical drive assembly may include a vertical drive motor, a vertical screw, a mounting block, a vertical drive chain, and a first follower wheel. The vertical screw, the vertical drive motor, and the first follower wheel may be fixed to the mounting block, respectively. A first gear may be coaxially mounted on an output shaft of the vertical drive motor, and the vertical drive chain may be meshed and connected with the first gear and the first follower wheel respectively. The manipulator mechanism may be socketed to a rod body of the vertical screw and is fixedly connected with the vertical drive chain.

In some embodiments, the drive system may include a two-dimensional linear slide mechanism. The two-dimensional linear slide mechanism may be connected to the vertical drive assembly to drive the vertical drive assembly to move in a horizontal and transverse direction and a horizontal and longitudinal direction.

In some embodiments, the two-dimensional linear slide mechanism may include a transverse drive motor, a transverse screw, a mounting plate, and a transverse rack belt. The transverse screw and the transverse rack belt may be fixed to the mounting plate respectively, the mounting block is socketed to the rod body of the transverse screw, the transverse drive motor may be fixed to the mounting plate, the output shaft of the transverse drive motor may be coaxially provided with a second gear, and the second gear may be engaged with the transverse rack belt.

In some embodiments, the two-dimensional linear slide mechanism includes a longitudinal drive motor, a longitudinal drive chain, a second follower wheel, and a guiding strip. The longitudinal drive motor, the guiding strip, and the second follower wheel are fixed on the mounting frame, a third gear is coaxially provided on an output shaft of the longitudinal drive motor, and the longitudinal drive chain is connected in mesh with the third gear and second follower wheel respectively; and the guiding strip is clipped on the mounting plate, and the mounting plate is connected to the longitudinal drive chain.

In some embodiments, the manipulator mechanism may include a pick-up arm and a steering mechanism. The pick-up arm may be configured to pick up and place the biological sample. The steering mechanism may include a rotating rack, a manipulator motor, and an offset guiding mechanism. The pick-up arm may be provided on the rotating rack, and the offset guiding mechanism may be connected with the rotating rack. The manipulator motor may be connected respectively to the rotating rack and the offset guiding mechanism for driving the rotating rack to rotate and for driving the offset guiding mechanism to move to drive the rotating rack to slide in a center axis direction during rotation.

In some embodiments, the cabinet may further include a sampling mechanism. The sampling mechanism may include a drive system and a manipulator mechanism for picking up and placing material. The drive system may drive the manipulator mechanism to move. The cabinet body may be fixed with class A shelves and class B shelves. The class A shelves may close to or abut at least one side wall of the cabinet body. The class B shelves may be set two by two adjacent to each other or close to each other and back to each other as a class B shelf set. Spacing channels for the manipulator mechanism to move may be provided on two sides of a width direction of the class B shelf set and one side of the class A shelves that is away from the side wall of the cabinet body. A width of each of the spacing channels may be greater than or equal to 0.5 times a width of each of the class A shelves or the class B shelves.

In some embodiments, the class A shelves may be close to or abut the cabinet body at two side walls or one side wall in a second straight line direction. A sum of the class A shelves and class B shelves may be an integer greater than or equal to 3. A single class A shelf may be designated as one shelf unit and a single class B shelf set as another shelf unit. All shelf units may be arranged in the second straight line direction with a spacing channel provided between adjacent shelf units, and a count of spacing channels may be (a count of class B shelves + 2)/2.

In some embodiments, the class A shelves may be close to or abut at least three side walls of the cabinet body.

In some embodiments, the class B shelves may be provided with a clearance channel for the manipulator mechanism to move and yield.

In some embodiments, the drive system may be the sampling mechanism. A guiding post clearance may be provided on the class B shelves or between the class B shelves and the cabinet body for a longitudinal guiding post of the sampling mechanism to move. The guiding post clearance may communicate with a clearance space, and the clearance space may be located at a top of the class B shelves.

In some embodiments, the cabinet may further include a material lifting device. The material lifting device may include a pick-up arm for picking up and placing material. The material lifting device may further include a steering mechanism, and the steering mechanism may include a base, a manipulator motor, and a rotating rack. The manipulator motor may be mounted on the base, and the manipulator motor may be configured to drive the rotating rack to rotate. The rotating rack may be provided with the pick-up arm. The material lifting device may further include a rotating center offset mechanism, the rotation center offset mechanism may be configured to drive the rotating center axis of the rotating rack to translate along a first linear direction when the rotating rack is moving, and the manipulator motor may be configured to drive the rotating center offset mechanism to move. The center axis direction of the rotating rack may be perpendicular to the first linear direction.

In some embodiments, the pick-up arm may include a sliding member for picking up and placing material, and the sliding member may be slidably connected with the rotating rack through a guide rail. The pick-up arm may further include a pick-up drive mechanism to drive the sliding member to move linearly along the guide rail.

In some embodiments, the cabinet may further include a mounting base. The mounting base may be configured to support the steering mechanism, and a side of the mounting base may be provided with a guiding hole for slidingly connecting a guiding post. When the rotating center axis translates in the first linear direction, the rotating center axis may move from a side close to the guiding hole to a side away from the guiding hole or moves from the side away from the guiding hole to the side close to the guiding hole.

In some embodiments, the cabinet may further include a mounting base. The mounting base may be configured to support the steering mechanism, and a side of the mounting base may be provided with a guiding hole for slidingly connecting a guiding post. The manipulator motor may be located on either side of the rotating rack in the center axis direction of the guiding hole.

In some embodiments, the rotating center offset mechanism may include a guiding assembly configured to guide the base to be slidably connected with the mounting base in the first linear direction.

In some embodiments, the rotating center offset mechanism may further include a connecting rod, one end of the connecting rod may be hinged to the base, and another end of the connecting rod may be hinged to the rotating rack.

In some embodiments, the rotating center offset mechanism may include a guiding assembly configured to guide the rotating center axis of the rotating rack to slide in the first linear direction.

In some embodiments, the guiding assembly may include a slide rail and a slide block. A center of the slide block may be rotationally connected to the rotating rack by a rotating shaft. A power output end of the manipulator motor may be connected to one end of an oscillating arm through a gear drive mechanism, another end of the oscillating arm may be movably connected to a guiding rod, and the guiding rod may be connected to the rotating rack. The base and the slide rail may be mounted on the mounting base, the mounting base may be provided with a guiding groove for guiding the guiding rod to move, and the guiding groove may be U-shaped.

In some embodiments, the rotating rack may be provided with a camera device and a light source adjacent to the camera device.

In some embodiments, the cabinet body may be provided with shelves, at least one side of the shelves in a width direction may be provided with a passageway for the material lifting device to move, and a length direction of the passageway may be parallel to the first linear direction.

In some embodiments, shelves may be provided within the cabinet, and a passageway may be provided between two adjacent shelves for the material lifting device to move. A length direction of the passageway may be parallel to the first linear direction. The passageway may be directly opposite a shipping port, and the shipping port may be located on a side of the mounting base away from the guiding hole. An orientation of the pick-up arm may be adjusted during rotation of the rotating rack, and the pick-up arm may be oriented in sequence toward one of the two adjacent shelves, the shipping port, and another one of the two adjacent shelves. The rotating center axis of the rotating rack may be located on the side close to the guiding hole when the pick-up arm is oriented toward either of the two adjacent shelves. The rotating center axis of the rotating rack may be located on the side away from the guiding hole when the pick-up arm facing the shipping port.

In some embodiments, the cabinet body may include an outer shell defining a shell cavity and an inner liner provided in the shell cavity, the inner liner may define an accommodating cavity, the outer shell may be provided with a first wiring port, the inner liner may be provided with a second wiring port, and the first wiring port and the second wiring port may have different positions in at least two directions. The accommodating cavity may be provided with a drive system, the drive system may be connected to one end of a cable, another end of the cable may extend out of the shell cavity after passing through the second wiring port and the first wiring port, and the cable may be configured to realize power supply and/or control of the drive system.

In some embodiments, a mounting mechanism may be fixed to an outer wall of the outer shell, the mounting mechanism may be provided with a control device, and the control device may be connected to another end of the cable.

In some embodiments, the first wiring port may be configured to place a first wiring box, and the second wiring port may be configured to place a second wiring box. The another end of the cable may pass through the second wiring port via the second wiring box, and the another end of the cable may pass through the first wiring port via the first wiring box.

### BRIEF DESCRIPTION OF THE DRAWINGS

The present disclosure is further illustrated in terms of exemplary embodiments. These exemplary embodiments are described in detail with reference to the drawings, in which like reference numerals represent similar structures or steps, and wherein:
FIG. 1 is a schematic diagram illustrating a low-temperature preservation cabinet for a biological sample according to some embodiments of the present disclosure;
FIG. 2 is a schematic diagram illustrating a state of a low-temperature preservation cabinet for a biological sample when a sampling mechanism is withdrawn from a cabinet body according to some embodiments of the present disclosure;
FIG. 3 is a schematic diagram illustrating a portion of a structure of a low-temperature preservation cabinet for a biological sample in the state shown in FIG. 2;
FIG. 4 is a schematic diagram illustrating a connection relationship between a support strip and a mounting frame of a low-temperature preservation cabinet for a biological sample according to some embodiments of the present disclosure;
FIG. 5 is a schematic diagram illustrating an internal structure of a low-temperature preservation cabinet for a biological sample according to some embodiments of the present disclosure;
FIG. 6 is a schematic diagram illustrating a side view of FIG. 5;
FIG. 7 is a schematic diagram illustrating an internal structure of a low-temperature preservation cabinet for a biological sample in another state according to some embodiments of the present disclosure;
FIG. 8 is a schematic diagram illustrating an internal structure of a low-temperature preservation cabinet for a biological sample from another view according to some embodiments of the present disclosure;
FIG. 9 is a schematic diagram illustrating an arrangement of class A shelves and class B shelves according to some embodiments of the present disclosure;
FIG. 10 is a schematic diagram illustrating another arrangement of class A shelves and class B shelves according to some embodiments of the present disclosure;
FIG. 11 is a front view of a low-temperature preservation cabinet for a biological sample according to some embodiments of the present disclosure;
FIG. 12 is a side view of a low-temperature preservation cabinet for a biological sample according to some embodiments of the present disclosure;
FIG. 13 is a three-dimensional view of a low-temperature preservation cabinet for a biological sample according to some embodiments of the present disclosure;
FIG. 14 is a schematic diagram illustrating gas flow in a low-temperature preservation cabinet for a biological sample according to some embodiments of the present disclosure;
FIG. 15 is a schematic diagram illustrating a structure of a low-temperature preservation cabinet for a biological sample without a drive system and shelves according to some embodiments of the present disclosure;
FIG. 16 is a schematic diagram illustrating the structure of the low-temperature preservation cabinet for the biological sample shown in FIG. 15 without a side panel of a cabinet body;
FIG. 17 is a schematic diagram illustrating the structure of the low-temperature preservation cabinet for the biological sample shown in FIG. 16 without a side cabinet;
FIG. 18 is a schematic diagram illustrating another view of the structure shown in FIG. 17;
FIG. 19 is a schematic diagram illustrating a first view of a manipulator mechanism according to some embodiments of the present disclosure;
FIG. 20 is a schematic diagram illustrating a second view of a manipulator mechanism according to some embodiments of the present disclosure;
FIG. 21 is a schematic diagram illustrating a first partial structure of a manipulator mechanism according to some embodiments of the present disclosure;
FIG. 22 is a schematic diagram illustrating a second partial structure of a manipulator mechanism according to some embodiments of the present disclosure;
FIG. 23 is a schematic diagram illustrating a third partial structure of a manipulator mechanism according to some embodiments of the present disclosure;
FIG. 24 is a schematic diagram illustrating a fourth partial structure of a manipulator mechanism according to some embodiments of the present disclosure;
FIG. 25 is a schematic diagram illustrating a structure of a manipulator mechanism according to another embodiment of the present disclosure;
FIG. 26 is a schematic diagram illustrating a structure of another view of a manipulator mechanism according to another embodiment of the present disclosure;
FIG. 27 is a schematic diagram illustrating a first view of a partial structure of a manipulator mechanism according to another embodiment of the present disclosure;
FIG. 28 is schematic diagram illustrating a second view of a partial structure of a manipulator mechanism according to another embodiment of the present disclosure;
FIG. 29 is schematic diagram illustrating a third view of a partial structure of a manipulator mechanism according to another embodiment of the present disclosure;
FIG. 30 is a schematic diagram illustrating another partial structure of a manipulator mechanism according to another embodiment of the present disclosure;
FIG. 31 is a schematic diagram illustrating a partial structure of a manipulator mechanism according to some embodiments of the present disclosure; and
FIG. 32 is a schematic diagram illustrating different states of circumferential rotation of a rotating rack of a manipulator mechanism according to some embodiments of the present disclosure.

Reference signs: 100 represents a cabinet body, 110 represents a cabinet door, 111 represents a sampling port, 112 represents a maintenance port, 120 represents a storage region, 130 represents an equipment region, 140 represents a temperature isolation region, 141 represents a temperature isolation device, 150 represents an outer shell, 151 represents a first wiring box, 160 represents an inner liner, 161 represents a second wiring box, 200 represents an evaporator, 300 represents a sampling mechanism, 310 represents a support strip, 311 represents a bump, 320 represents a mounting frame, 321 represents a positioning pin, 411 represents a vertical drive motor, 412 represents a vertical screw, 413 represents a mounting block, 414 represents a vertical drive chain, 415 represents a first follower wheel, 421 represents a transverse drive motor, 423 represents a mounting plate, 424 represents a transverse rack belt, 431 represents a longitude drive motor, 432 represents a vertical drive chain, 433 represents a second follower wheel, 434 represents a guiding strip, 500 represents a manipulator mechanism, 510 represents a mounting base, 520 represents a pick-up arm, 521 represents a sliding member, 522 represents a guide rail, 523 represents a pick-up drive mechanism, 524 represents a limiting protrusion, 530 represents a base, 540 represents a manipulator motor, 541 represents an active gear, 542 represents a driven gear, 550 represents a rotating rack, 551 represents a rotating shaft, 560 represents a connecting rod, 571 represents an oscillating arm, 572 represents a guiding rod, 573 represents a slide block, 574 represents a guiding groove, 575 represents a guiding hole, 576 represents a sliding rail, 577 represents a guiding holes, 580 represents a guiding assembly, 591 represents a camera device, 592 represents a light source, 593 represents a laser range finder, 600 represents a mounting mechanism, 700 represents a compressor, 801 represents a shelf, 802 represents a passageway, 810 represents a first rack body, 820 represents a second rack body, 830 represents a first travel channel, 840 represents a second travel channel, 850 represents a clearance channel, 860 represents a class A shelf, 870 represents a class B shelf, 880 represents a spacing channel, 900 represents a mounting box, and 1000 represents a low-temperature preservation cabinet for a biological sample.

### DETAILED DESCRIPTION

To more clearly illustrate the technical solutions related to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings.

It should be understood that "system", "equipment", "device", "part" and/or "member" as used herein is a manner used to distinguish different components, elements, parts, members, sections, or assemblies at different levels. However, if other words serve the same purpose, the words may be replaced by other expressions.

In the absence of special instructions, the technical terms used in the present disclosure to describe the components, elements, etc., do not specifically refer to the singular, but may also include the plural. In general, the terms "comprise", "comprises", "comprising", "include", "includes", and/or "including", merely prompt to include operations and elements that have been clearly identified, and these operations and elements do not constitute an exclusive listing. The methods or devices may also include other operations or elements.

In the description of the present disclosure, it is to be understood that the orientation or positional relationships indicated involving orientation descriptions, such as up, down, forward, backward, left, right, or the like, are based on those shown in the accompanying drawings, and are intended only for the purpose of facilitating the description of the present disclosure and simplifying the description, and is not intended to indicate or imply that the device or element referred to must have a particular orientation, be constructed and operated in a particular orientation, and therefore is not to be construed as a limitation of the present disclosure. In the description of the present disclosure, unless otherwise expressly limited, the words "setup", "mounting", "connection", or the like, shall be broadly construed, and a person skilled in the art may reasonably determine the specific meaning of the words in the present disclosure by taking into account the specific contents of the technical solution.

The low-temperature preservation cabinet for a biological sample is a professional refrigerator that utilizes a low-temperature environment (with a temperature not higher than minus 70°) for the storage of clinical biological samples, etc. As the low-temperature preservation cabinet for the biological sample provides a preservation temperature not higher than -70°C. A drive system set up to drive a manipulator in the preservation cabinet should have good low temperature resistance. However, the low-temperature drive system is expensive, which raises the cost of the device. Therefore, it provides a research direction to those skilled in the art to develop a new type of preservation cabinet for the biological sample to overcome the above problems.

One or more embodiments of the present disclosure provide a low-temperature preservation cabinet for a biological sample with temperature zones, which eliminates the need to set the drive system at a cryogenic temperature no higher than -70 °C, thereby reducing costs. In some embodiments, the low-temperature preservation cabinet for the biological sample includes a cabinet body and a temperature isolation region. The cabinet body is provided with a storage region and an equipment region, and the storage region is configured to store a biological sample. The temperature isolation region is provided between the storage region and the equipment region, with the temperature of the storage region being lower than the temperature of the equipment region.

Some embodiments of the present disclosure provide a low-temperature preservation cabinet for a biological sample with temperature zones including a cabinet body 100, a sampling mechanism 300, and a refrigeration mechanism. In some embodiments, the cabinet body 100 is provided with a sampling port 111 (which may be opened and closed), and the cabinet body 100 is provided with a storage region 120 (or referred to as a sample layer) and an equipment region 130 (or referred to as an equipment layer). The storage region is provided with a biological sample rack (e.g., a first rack body 810 and a second rack body 820) and a sampling travel channel (e.g., a first travel channel 830 and a second travel channel 840). The biological sample rack is configured to place the biological sample. In some embodiments, a gap or temperature isolation device 141 is provided between the storage region and the equipment region. In some embodiments, a temperature isolation region 140 is provided between the storage region and the equipment region, and a gap or the temperature isolation device 141 is located in the temperature isolation region 140. In some embodiments, the sampling mechanism 300 includes a manipulator mechanism 500 and a drive system. The drive system is located in the equipment region, and the drive system is configured to drive the manipulator mechanism 500 to move in the sampling travel channel and the manipulator mechanism 500 is configured to pick up and place the biological sample. In some embodiments, the refrigeration mechanism is configured to control the temperature of the storage region to be within a temperature range not higher than -70°C and the temperature of the equipment region to be within a temperature range not lower than -50°C. In some embodiments, the refrigeration mechanism is configured to control the temperature of the storage region to be within a temperature range not higher than -65°C and the temperature of the equipment region to be within a temperature range not lower than -55°C. In some embodiments, the refrigeration mechanism is configured to control the temperature of the storage region to be within a temperature range not higher than -75°C and the temperature of the equipment region to be within a temperature range not lower than -45°C. In some embodiments, the refrigeration mechanism may be configured to control a temperature difference between the storage region and the equipment region to be within a temperature range not less than 10°C.

Some embodiments of the present disclosure implement temperature zoning within the cabinet body 100, setting the biological sample in a low-temperature region and the drive system in a medium-temperature region, thereby reducing the need for cold-resistant performance of the drive system, and lowering the cost of the device.

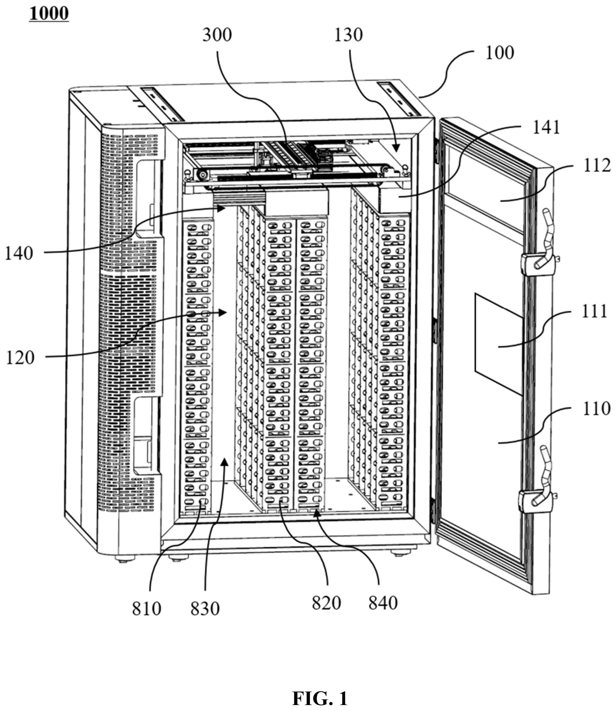

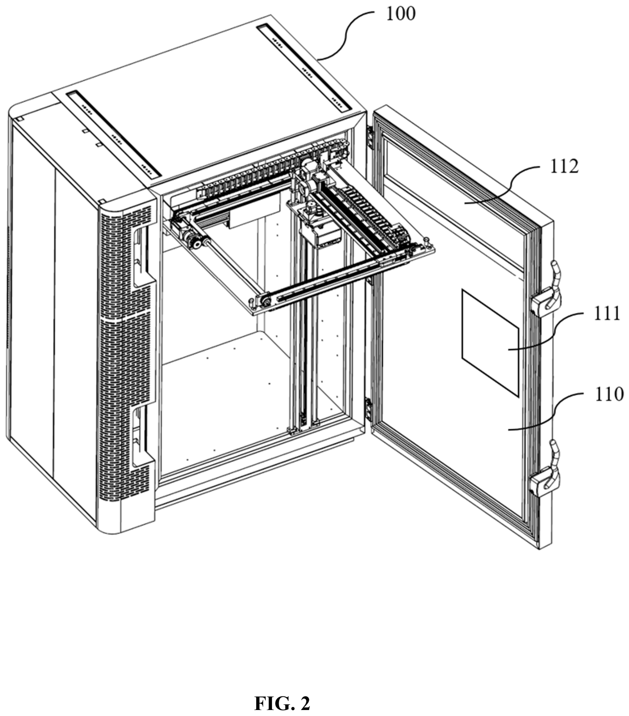

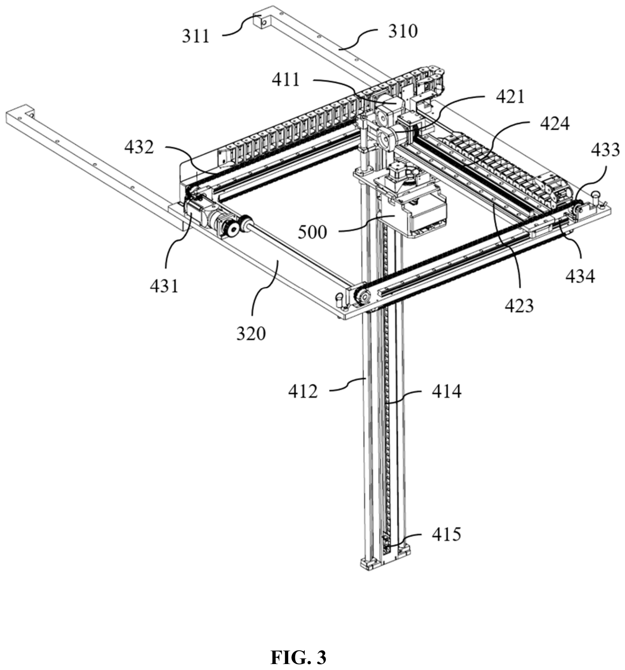

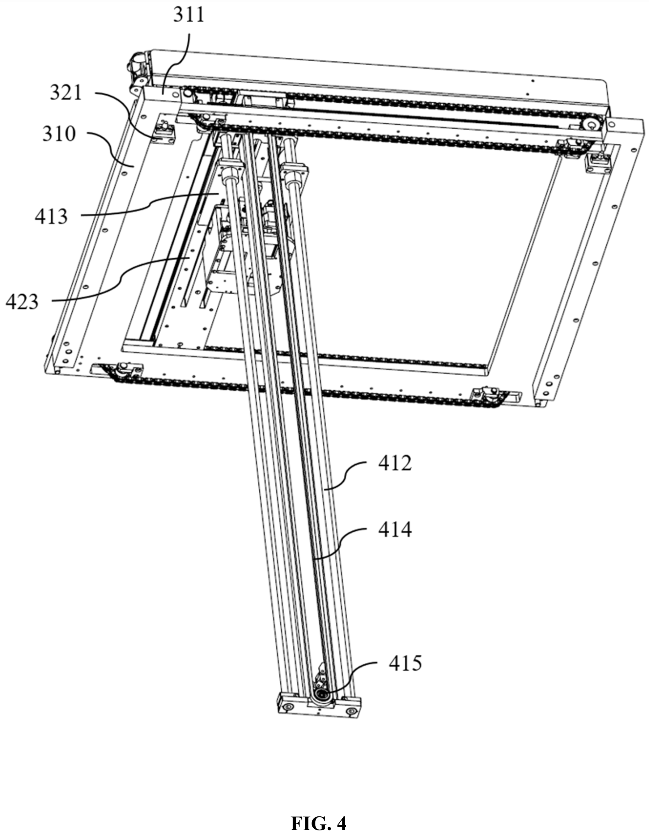

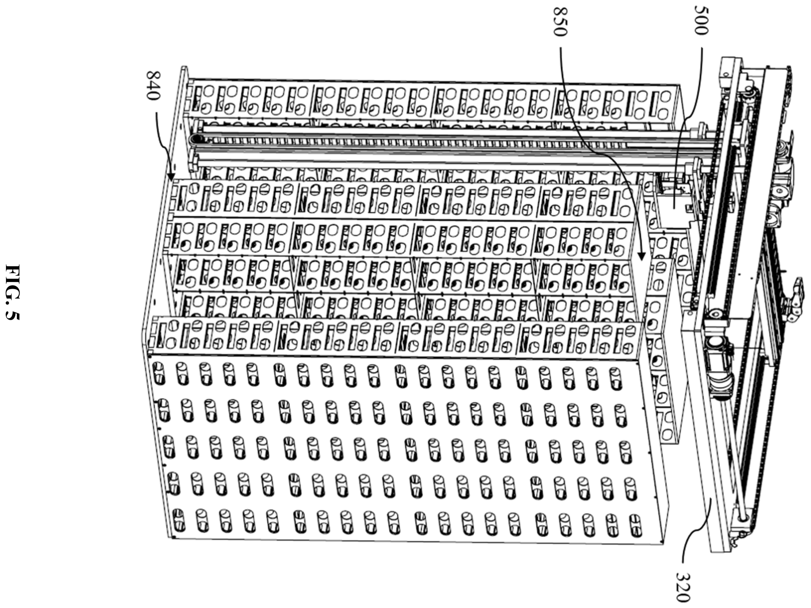

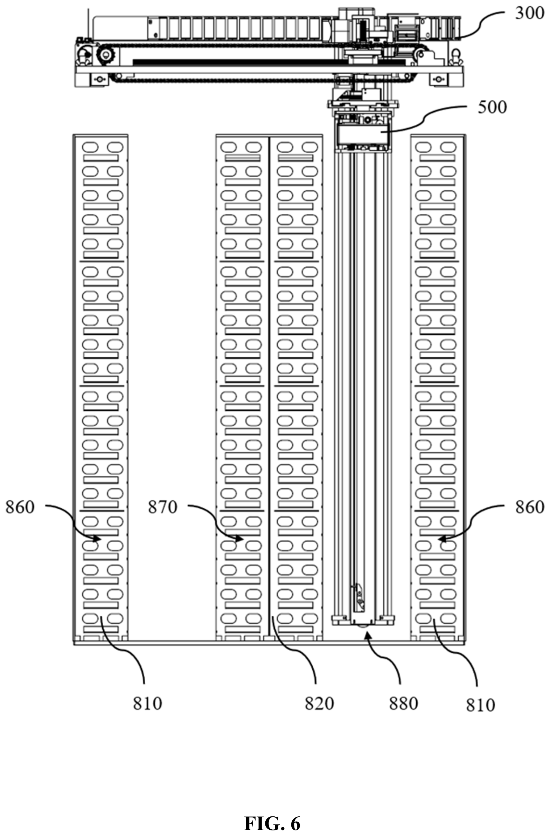

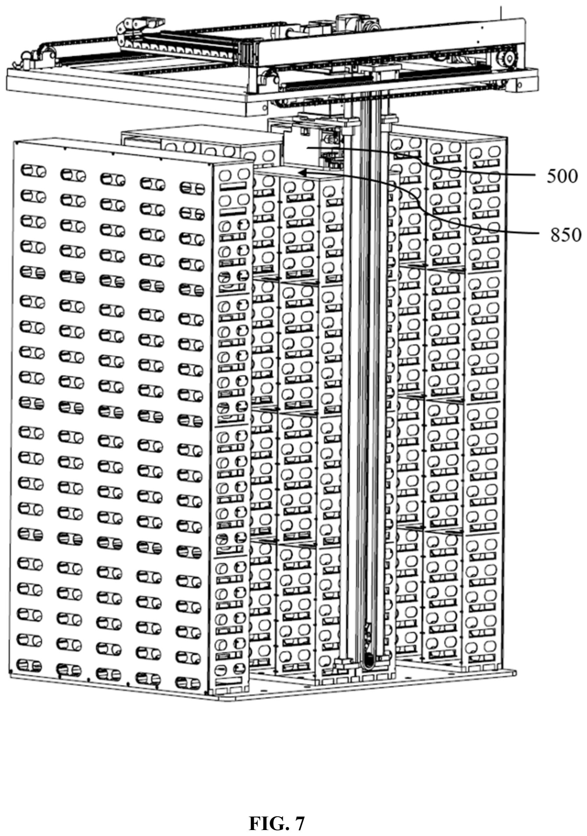

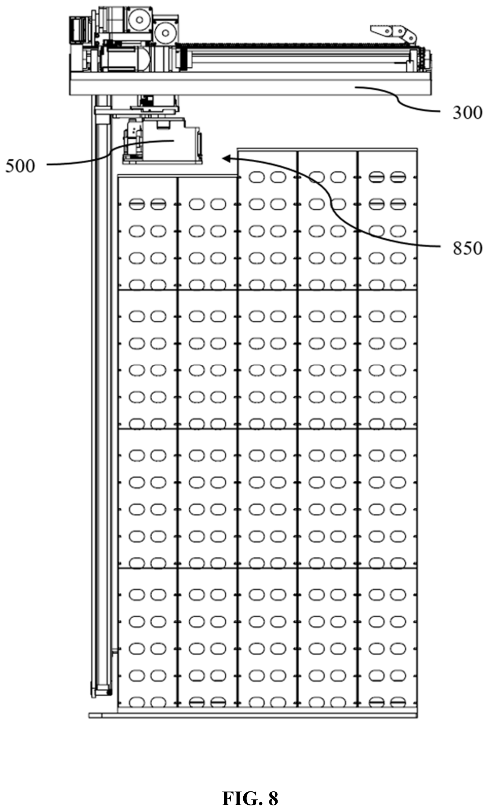

FIG. 1 is a schematic diagram illustrating a low-temperature preservation cabinet for a biological sample according to some embodiments of the present disclosure. FIG. 2 is a schematic diagram illustrating a state of a low-temperature preservation cabinet for a biological sample when a sampling mechanism is withdrawn from a cabinet body according to some embodiments of the present disclosure. FIG.3 is a schematic diagram illustrating a portion of a structure of a low-temperature preservation cabinet for a biological sample in the state shown in FIG. 2. FIG. 4 is a schematic diagram illustrating a connection relationship between a support strip and a mounting frame of a low-temperature preservation cabinet for a biological sample according to some embodiments of the present disclosure. FIG. 5 is a schematic diagram illustrating an internal structure of a low-temperature preservation cabinet for a biological sample according to some embodiments of the present disclosure. FIG. 6 is a schematic diagram illustrating a side view of FIG. 5. FIG. 7 is a schematic diagram illustrating an internal structure of a low-temperature preservation cabinet for a biological sample in another state according to some embodiments of the present disclosure. FIG. 8 is a schematic diagram illustrating an internal structure of a low-temperature preservation cabinet for a biological sample from another view according to some embodiments of the present disclosure.

In some embodiments, referring to FIGs. 1-8, a low-temperature preservation cabinet 1000 for a biological sample with temperature zones (referred to as the preservation cabinet 1000 hereinafter) may include a cabinet body 100, a sampling mechanism 300, and a refrigeration mechanism (not shown in the figures).

In some embodiments, the cabinet body 100 is provided with a sampling port 111, and the cabinet body 100 is provided with a storage region 120 and an equipment region 130. In some embodiments, the equipment region is provided above the storage region. The storage region is provided with a biological sample rack and a sampling walking channel. The biological sample rack is configured to place a biological sample, and a temperature isolation device 141 is provided between the storage region and the equipment region. In some embodiments, the temperature isolation device 141 is made of temperature isolation foam (in other embodiments, the temperature isolation device may also be made of other temperature isolation materials such as foam), and the temperature isolation foam is fixed to the top of the storage region as a unit (and, in other embodiments, may be removably connected). By fixing the temperature isolation foam to the top of the storage region as a unit, it is possible to prevent the temperature isolation foam from deflecting toward the storage region or the equipment region. In some embodiments, the temperature isolation device 141 is provided with a channel that connects the storage region and the equipment region. In some embodiments, the storage region 120 is covered with an insulating layer around the storage region 120 and at the bottom, to facilitate the formation of different temperature zones of the inner cavity of the cabinet body 100 to avoid the ambient temperature in which the equipment region 130 is located from being too low for the cause of equipment failure.

In some embodiments, the sampling mechanism 300 includes a manipulator mechanism 500 and a drive system. The drive system is disposed in the equipment region and the drive system is configured to drive the manipulator mechanism 500 to move in the sampling travel channel. The manipulator mechanism 500 is configured to pick up and place the biological sample. In some embodiments, the sampling mechanism 300 is fully automated, and one end of the manipulator mechanism 500 passes through the channel to connect to the drive system.

In some embodiments, the refrigeration mechanism (which may utilize a plurality of refrigerators such as compression, semiconductor, etc.) is configured to control the temperature of the storage region to be within a temperature range not higher than -70°C, and the temperature of the equipment region to be within a temperature range not lower than -50°C. The refrigeration mechanism is preferably provided away from the equipment region. In some embodiments, the refrigeration mechanism is provided near the bottom of the storage region. By providing the refrigeration mechanism near the bottom of the storage region, it is possible to allow cold air to pass through from the bottom of the storage region, thereby facilitating the cooling of the storage region. By locating the refrigeration mechanism away from the equipment region, it is possible to reduce the cooling effect of the refrigeration mechanism on the equipment region, thereby controlling the temperature of the equipment region to be higher than the temperature of the storage region.

In some embodiments, a temperature isolation device is not provided between the storage region and the equipment region, while a gap layer is provided between the storage region and the equipment region. In some embodiments, the height of the gap layer takes any value between 80 mm and 200 mm. The present disclosure relies on the property of the air filled in the gap layer to act as a poor conductor of heat to create a temperature step between the storage region and the equipment region. Therefore, the overall design height of the cabinet body 100 may be higher relative to the design height of the cabinet body 100 with a temperature isolation device under the premise not change in the storage region and the equipment region. However, the advantage is that there is no need to set up a temperature isolation device, which has high installation, production efficiency, and cost savings.

In some alternative embodiments, the equipment region may be located directly below the storage region. The top of the biological sample rack in the storage region is fixed to the inner top wall of the cabinet body 100. In some alternative embodiments, the storage region and the equipment region are lined up in a horizontal direction within the cabinet body 100. Specifically, the equipment region is disposed on the horizontal right side of the cabinet body 100, the storage region is disposed on the horizontal left side of the cabinet body 100, and one end of the biological sample rack is fixed to the left side wall of the cabinet body 100. In some alternative embodiments, the equipment region and the storage region may be provided in a wrap-around or layered configuration. For example, the equipment region may be provided around the storage region. As another example, the storage region may be set around the equipment region.

In some embodiments, the biological sample and other samples in the prior art are usually stored in a biological sample freezer, which is generally provided with shelves (which may also be referred to as a biological sample rack or a cryopreservation rack, where the biological sample may be placed in a cryopreservation tube or a cryopreservation bag, where the cryopreservation tube or the cryopreservation bag is placed in a cryopreservation box, the cryopreservation rack store the cryopreservation box for storing the biological sample, the cryopreservation rack may be defined with one or plurality of rack cavities, and each of which may hold one or more cryopreservation boxes) and a manipulator mechanism for traveling between adjacent shelves and for picking up and placing materials. The biological sample freezer in the related art is only individually provided with shelves adjacent to, or immediately adjacent to, the inner wall of the cabinet body, which is not conducive to assuring increased placement of items within the cabinet body.

Some embodiments of the present disclosure provide a low-temperature preservation cabinet for a biological sample (or referred to as a biological sample freezer) including a cabinet body, and further including a sampling mechanism (or referred to as a pick-and-place mechanism). The sampling mechanism includes a drive system and a manipulator mechanism 500 for picking up and placing materials, and the drive system drives the manipulator mechanism 500 to move. In some embodiments, the cabinet body is fixed with class A shelves 860 and class B shelves 870 inside the cabinet body. The class A shelves 860 close to or abut at least one side wall of the cabinet, and the class B shelves 870 are provided close to or abut each other and back to each other as a class B shelf set. The class B shelf set is provided with spacing channels 880 on two sides of the width direction and on one side of the class A shelves away from a side wall of the cabinet body. In some embodiments, the width of a spacing channel is greater than or equal to 0.5 times the width of the class A shelves 860 or 0.5 times the width of the class B shelves 870. Some embodiments of the present disclosure optimize shelf arrangement within the cabinet body through a combination of class A shelves 860 adjacent to class B shelves 870, which in turn achieves a high storage capacity within the cabinet body.

In some embodiments, the width of the spacing channel is less than or equal to two times the width of the class A shelf 860 or two times the class B shelf 870 to ensure the storage density. In some embodiments, the width of the spacing channel is less than or equal to 1.2 times the width of the class A shelf 860 or 1.2 times the width of the class B shelf 870. "Abut" means that a distance between the two is 0, and "adjacent" means that the distance between the two is less than or equal to a set value, which may be any value within a range of 0-10 cm, for example, 5 cm. The back-to-back setup means that the sides of two class B shelves 870 for pick up and place the material are opposite. That is, the sides of the two class B shelves 870 that is away from the side used for pick up and place the material is close to each other. The width of the spacing channel refers to a distance between the class B shelf set and the adjacent class A shelf or class B shelf set. That is, the width direction of the spacing channel is a direction in which the material on the shelves is picked up and placed. For example, the width direction of the spacing channel is a left-right direction. The left or right side of a class A shelf or an adjacent class B shelf set may be used for picking up and placing the material. The length direction of the spacing channel is a front-rear direction. The manipulator mechanism may move forward and backward, up and down to pick up and place the material at different locations on the shelves. In some embodiments, the shelves for picking up and placing the material from the left or the right may be suitable for storing more types of cryopreservation boxes. Furthermore, it is more convenient to pick up and place the material from the left or the right as compared to picking up and placing the material from the upper part. In some alternative embodiments, the openings for picking up and placing the material may be located above the shelves, the cryopreservation boxes may be placed directly in the shelf cavities, and one or more cryopreservation boxes may be placed in each of the shelf cavities, and then the manipulator mechanism may directly take the cryopreservation boxes from the shelf cavities. In some embodiments, the shelf may be provided with a plurality of storage cavities, and one or more cryopreservation boxes may be stored in each storage cavity. The cryopreservation boxes may store the biological sample. For example, two cryopreservation boxes may be stored in each storage cavity, with the two cryopreservation boxes being set side by side left and right. By enabling a single storage cavity to store two or more cryopreservation boxes, the storage capacity of the cabinet body 100 may be effectively enhanced.

In some embodiments, the shelves may be arranged in a circular or cylindrical shape (the shelves may be rotated along a circular trajectory, in other embodiments the shelves may be rotated along other shapes such as rectangular, oval, etc., and the rotating axis of the shelves may be horizontal, or vertical, or along other directions). In some embodiments, the storage region may be provided around the equipment region, at which time the shelves may be arranged in a ring shape, and the sampling mechanism 300 may be provided in the middle of the ring-shaped shelves and pick up and place the material from the side of the ring-shaped shelves. In some embodiments, the equipment region may be provided around the storage region, at which time the shelves may be arranged in a cylindrical shape, and the sampling mechanism 300 may be provided around the cylindrical shelves and pick up and place the material from the side of the cylindrical shelves. In some embodiments, the shelves may be provided in a single ring or multiple rings when the shelves are arranged in a ring or cylindrical shape. When the shelves are provided in multiple rings, two or more cryopreservation boxes may be stored in a single storage cavity on the shelves.

In some embodiments, the manipulator mechanism may pick up and place the cryopreservation boxes from a side of the cryopreservation rack. The cryopreservation boxes may be placed directly in the shelf cavity, and one or more cryopreservation boxes may be placed in each shelf cavity, the manipulator mechanism may pick up and place the cryopreservation boxes directly from the shelf cavity. The cryopreservation boxes may also be provided on the shelf cavity by a transit member, which may be a sub-shelf body, a drawer, a tray, or the like. One or more transit members may be provided on each shelf cavity, for example, a plurality of transit members provided along a direction of the manipulator mechanism. Each transit member may be used for placing one or more cryopreservation boxes, so that when the manipulator mechanism picks up and places the cryopreservation boxes, the manipulator mechanism or a transit member transfer mechanism may first move the transit members out of the shelf cavity, and then the manipulator mechanism picks up and places the cryopreservation boxes.

In other embodiments, the cryopreservation rack may be lifted up, for example, by a manipulator mechanism or a rack lifting mechanism, etc., and one cryopreservation rack or a plurality of cryopreservation racks arranged along an upward and downward direction may be disposed at the same horizontal position, and each of the cryopreservation racks may be placed with one or a plurality of cryopreservation boxes. When the cryopreservation rack is lifted up, the manipulator mechanism then carries out the operation of picking up and placing the cryopreservation boxes on the cryopreservation rack that is lifted up. The one or more cryopreservation racks located at the same horizontal position may be provided with a limit structure, and the limit structure is configured to limit the position and/or the movement direction of the corresponding cryopreservation racks, for example, the limit structure may be a limit rod, a limit sleeve, or the like. The limit sleeve may define a sleeve cavity for accommodating the corresponding cryopreservation rack.

In some embodiments, the following options are possible with respect to the arrangement of shelves.

Option 1: class A shelves are adjacent to or abut the cabinet body on two side walls or one side wall along a second linear direction. For example, with the second linear direction as a left-right direction, the class A shelves are adjacent to or abut at least one of a left side wall and a right side wall of the cabinet body. In a horizontal cross-section of the cabinet body, a length of the cabinet body in the second linear direction may be greater than a length of the cabinet in a direction perpendicular to the second linear direction. The length of the cabinet in the left-right direction may be greater than the length of the cabinet body in the front-rear direction, i.e., the class A shelves may tightly abut two side walls on a side with shorter length of the cabinet body.

A sum of a count of class A shelves 860 and class B shelves 870 is an integer greater than or equal to 3. A single class A shelf as a shelf unit, a single class B shelf set as another shelf unit, the single class B shelf set is a set of class B shelves. All shelf units are arranged along the second linear direction, and there are spacing channels between adjacent shelf units. A count of spacing lanes is (the count of class B shelves 870+2)/2.

In some embodiments, the specific shelf arrangement scheme may be as follows.

There is one class B shelf set, and there are a class A shelf, a spacing channel, a class B shelf set, a spacing channel, and a class A shelf in the cabinet body in sequence along the second linear direction. The spacing channel is set to 2, the class A shelf is set to 2, and the class B shelves are set to 2. The width of the spacing channel is 0.5 times the width of each of the class A shelf. At this time, the storage density (projected area utilization) is 4/5.

There is one class B shelf set, and there are a class A shelf, a spacing channel, a class B shelf set, a spacing channel, and a class A shelf, in the cabinet body in sequence along the second linear direction. The spacing channel is set to 2, the class A shelf is set to 2, and the class B shelves are set to 2. The width of the spacing channel is 0.8 times the width of each of the class A shelf. At this time, the storage density (projected area utilization) is 5/7.

There are two class B shelf sets, and there is a class A shelf, a spacing channel, a class B shelf set, a spacing channel, a class B shelf set, a spacing channel, and a class A shelf in the cabinet in sequence along the second linear direction. The spacing channel is set to 3, the class A shelf is set to 2, and the class B shelves are set to 4. The width of the spacing channel is as the same as the width of each of the class A shelves. At this time, the storage density (projected area utilization) is 2/3.

There are three class B shelf sets, and there is a class A shelf, a spacing channel, a class B shelf set, a spacing channel, a class B shelf set, a spacing channel, a class B shelf set, a spacing channel, and a class A shelf in the cabinet body in sequence along the second linear direction. The spacing channel is set to 4, the class A shelf is set to 2, and the class B shelves are set to 6. The width of the spacing channel is 1.2 times the width of each of the class A shelves. At this time, the storage density (projected area utilization) is 5/8.

Option 2: class A shelves are adjacent to or abut at least three side walls of the cabinet body; the drive system includes a lifting mechanism used to drive the manipulator mechanism 500 to move up and down and a rotating mechanism that drives the lifting mechanism to rotate in a circumferential direction, and a rotating end of the rotating mechanism is connected to the lifting mechanism; and the spacing channel is a space for the lifting mechanism to rotate.

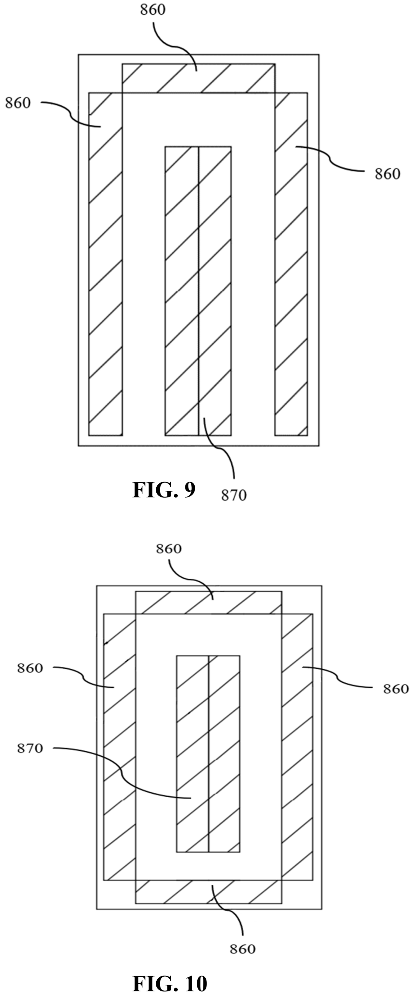

FIG. 9 is a schematic diagram illustrating an arrangement of class A shelves and class B shelves according to some embodiments of the present disclosure. FIG. 10 is a schematic diagram illustrating another arrangement of class A shelves and class B shelves according to some embodiments of the present disclosure. In some embodiments, as shown in FIG. 9, with respect to the arrangement scheme of the shelves, it is possible that the class A shelves are adjacent to or abutting the three side walls of the cabinet body, and that the count of the class B shelf set is 1. As shown in FIG. 10, the class A shelves are adjacent to or abutting four side walls of the cabinet body, and the count of the class B shelf set is 1.

In some embodiments, the sampling mechanism may perform multi-directional pick up and place operations. Specifically, the drive system may be magnetically controlled. Alternatively, the drive system may be a sampling mechanism 300 (or referred to as a three-axis linear slide mechanism). The three-axis linear slide mechanism includes an X-direction linear slide mechanism that slides along the second linear direction, a Y-direction linear slide mechanism that slides along the first linear direction, and a lifting mechanism that raises and lowers along a longitudinal direction. The first linear direction is perpendicular to the second linear direction. The second linear direction and the first linear direction are perpendicular to the longitudinal direction.

In some embodiments, the manipulator mechanism 500 is a steerable manipulator mechanism 500, a retractable manipulator mechanism 500, or a steerable and retractable manipulator mechanism 500.

In some embodiments, when the width of the spacing channel is equal to 0.5 times the width of the class A shelf 860 or 0.5 times the width of the class B shelf 870, two rows of material may be placed inner and outer of the class A shelf or the class B shelf. After the manipulator mechanism removes the material adjacent to the manipulator mechanism by telescoping, the material far away from the manipulator mechanism may be removed by the continued elongation of the manipulator mechanism at the next pickup.

In some embodiments, the class B shelf 870 is provided with a clearance channel 850 (or clearance space) for the manipulator mechanism 500 to move and yield. The clearance channel 850 is a space for the manipulator mechanism 500 to cross adjacent spacing channels. The clearance channel 850 may be a channel or a notch. When the clearance channel 850 is a channel, the channel may run through adjacent spacing channels. When the clearance channel 850 is a notch, the notch is located at the top of the end portion of the length direction of the class B shelf 870. By setting the clearance channel 850 as a notch, the size of the clearance channel 850 on the class B shelf 870 is reduced, increasing the placement rate of the material on the shelf and increasing the number of material placements with respect to setting the clearance channel 850 as a channel. The manipulator mechanism 500 may be raised to the highest point of the drive system when crossing adjacent spacing channels and passes the notch to proceed to cross the adjacent spacing channels. In some embodiments, the manipulator mechanism 500 may be parked at the clearance channel 850 when the manipulator mechanism 500 is not performing sampling work, which on the one hand may avoid the low temperature of the storage region from affecting the manipulator mechanism, and on the other hand, the manipulator mechanism 500 may move faster to any shelf for sampling when it receives a sampling command. In addition, the clearance channel 850 may be provided at the rear of the class B shelf 870, so that when the manipulator mechanism 500 is not carrying out the sampling work, a pick-up arm of the manipulator mechanism 500 may be oriented toward a shipping port. Thus, when the manipulator mechanism is ready to take samples, it only needs to be turned 90 degrees to the left or the right, thereby enhancing the sampling efficiency.

In some embodiments, the drive system is a three-axis linear slide mechanism. A guiding post clearance is provided on the class B shelf or between the class B shelf and the cabinet body for a longitudinal guiding post of the three-axis linear slide mechanism to move. The guiding post clearance communicates with the clearance channel 850, and the clearance channel 850 is located at a top of the class B shelf. In some embodiments, the longitudinal guiding post may be a vertical screw 412, and the guiding post clearance may be a second travel channel 840.

In some embodiments, the drive system includes a lifting mechanism for driving the manipulator mechanism 500 to lift. The lifting mechanism includes a longitudinal guiding column, and the manipulator mechanism 500 is provided on the longitudinal guiding column and may be lifted and lowered along the longitudinal guiding column. The spacing channel is used for the longitudinal guiding post to avoid. The longitudinal guiding post moves in the spacing channel when the drive system drives the manipulator mechanism 500 to move in the spacing channel.

In some embodiments, a maintenance port (which may be opened and closed) is provided on a side wall of the cabinet body, with the maintenance port facing the spacing channel. The longitudinal guiding post is mounted on a side of the manipulator mechanism 500 away from the maintenance port. Preferably, the cabinet body is provided with a maintenance port on a side wall in the first linear direction, the maintenance port being directly opposite the spacing channel. The first linear direction is perpendicular to the second linear direction. There are at least two longitudinal guiding posts, all of the longitudinal guiding posts being disposed along the second linear direction.

In some embodiments, at least a portion of the shelves may be movable relative to the cabinet body. For example, a class B shelf 870 disposed between two class A shelves may be movable with respect to the cabinet body. The class B shelf 870 may move to allow the class B shelf to be closer to one of the class A shelves on one side of the class B shelf and to form a spacing channel with the class A shelf on the other side, or to make the class B shelve close to the class A shelves on the other side and form a spacing channel between the class A shelves on one side. The storage capacity of the cabinet body 100 may be further enhanced by setting the class B shelf to be able to move relative to the cabinet body.

In some embodiments, the sampling mechanism 300 may include two and more manipulator mechanisms 500. For example, one sampling mechanism 300 may be provided in each spacing channel. By providing two and more manipulator mechanisms 500, it is possible to efficiently improve the efficiency of picking up and placing the material.

In some embodiments, the refrigerator, in order to maintain a low-temperature environment to the maximum extent, generally employs a structure in which a manipulator removes a biological sample from the cabinet body and moves it to a sampling port to achieve the sample outlet. When the manipulator and its drive system malfunction, the existing structure of the manipulator and the drive system are mounted on the cabinet body and are not easy to dismantle, which results in a long time of opening the door of the cabinet for repairing the equipment. This results in a large loss of cool air in the cabinet body, which is not conducive to the cooling and preservation of the biological sample, especially a clinical biological sample. Therefore, it provides a research direction to those skilled in the art to develop a new type of preservation cabinet for the biological sample to overcome the above problems.

In some embodiments, in order to provide quick removal of the sampling mechanism 300 for servicing and to reduce the loss of cold air caused by servicing, an accommodating cavity of the cabinet body 100 (divided into a storage region and an equipment region) has an opening, the opening being opened and closed by the cabinet door 110, and the cabinet door 110 is further provided with a maintenance port 112 (which may be opened and closed). In some embodiments, the cabinet door may be connected to a lateral side of the cabinet body, such as a left or right side, i.e., the cabinet door 110 may be a side door. In some embodiments, the sampling mechanism 300 includes a mounting frame 320, a drive system is mounted to the mounting frame 320, and the mounting frame 320, the drive system, and the manipulator mechanism are configured to slide and pull out the maintenance port simultaneously. In some embodiments, support strips 310 are symmetrically provided on opposite side walls of the cabinet body 100, the support strips 310 extend toward the maintenance port (or so-called fixing port), the mounting frame 320 is a rectangular frame, and two sides of the mounting frame 320 are placed on the support strips 310. By adopting this technical solution, when maintenance is required, dragging the mounting frame 320 causes the mounting frame 320 to be slid and pulled along the support strips 310, driving the drive system and the manipulator mechanism 500 to synchronously move out of the cabinet body 100 from the maintenance port. In practice, it is also possible to provide a sliding groove on the support strip 310s (it is also possible to provide the sliding groove directly on the inner wall of the cabinet body without the support strips 310), and to provide a roller (it is understood that the rolling of such a roller is a way to realize the sliding of the mounting frame 320 or the like, with respect to the maintenance port) or a slide block corresponding to the sliding groove on the mounting frame 320, so as to realize the rolling pulling of the mounting frame 320 along the support strips 310.

In some embodiments, one end of each support strip 310 back away from the maintenance port each support strip 310 is provided with a bump 311 perpendicular to the support strip 310, each bump 311 is provided with a positioning groove, and the mounting frame 320 is provided with a positioning pin 321 corresponding to the positioning groove. By adopting this technical solution, during the process of loading the overhauled drive system and the manipulator mechanism 500 into the cabinet body 100, through the cooperation of the positioning groove and the positioning pin 321, it is possible to realize that the mounting frame 320 and the support strip 310 may be quickly installed and positioned.

Some embodiments of the present disclosure provide a low-temperature preservation cabinet 1000 for a biological sample for easy maintenance, which may realize the rapid sliding of the robot mechanism 500 and the drive system to take out the cabinet body 100, and then satisfy the repair of the cabinet, reduce the maintenance process caused by the loss of cold air, and ensure the effect of low-temperature storage of the biological sample.

In some embodiments, a preservation cabinet for a biological sample, which is easy for maintenance, includes a cabinet body 100. The cabinet body 100 is provided with a maintenance port 112 (which may be opened and closed), for example, a cabinet door 110 is provided on a side wall of the cabinet body 100, the cabinet door 110 is provided with a maintenance port (in other embodiments, the maintenance port may be provided at a location on the cabinet body 100 other than the cabinet door 110, for example, a side wall, etc.), and the cabinet door 110 may further be provided with a sampling port 111. The cabinet body 100 is provided with a storage region and an equipment region, e.g., the cabinet body 100 includes a storage region and an equipment region disposed above the storage region (in other embodiments, the equipment region may be disposed in other directions of the storage region).

The equipment region includes a mounting frame 320. The mounting frame 320 is fixedly connected to a drive system, the drive system is connected to a manipulator mechanism 500 for driving the manipulator mechanism 500 to move in a sampling travel channel, and the manipulator mechanism 500 is configured to pick up and place the biological sample. The mounting frame 320 is configured to be slidable to be pulled out of the maintenance port, so that when maintenance is required, dragging the mounting frame 320 enables the mounting frame 320 to drive the drive system and the manipulator mechanism 500 to move out of the cabinet body 100 from the maintenance port simultaneously.

In some embodiments, the drive system includes a vertical drive motor 411, a transverse drive motor 421, a longitudinal drive motor 431, and a three-axis drive mechanism connected to the mounting frame 320. The three-axis drive mechanism is connected to the manipulator mechanism 500, the vertical drive motor 411, the transverse drive motor 421, and the longitudinal drive motor 431, respectively. The vertical drive motor 411 transmits and drives the manipulator mechanism in the vertical direction through the three-axis transmission mechanism. The transverse drive motor 421 transmits and drives the manipulator mechanism in the horizontal and transverse direction through the three-axis transmission mechanism. The longitudinal drive motor 431 transmits and drives the manipulator mechanism to move in the horizontal and longitudinal direction through the three-axis drive mechanism.

Specifically, the drive system includes a vertical drive assembly and a two-dimensional linear slide mechanism. The vertical drive assembly includes a vertical drive motor 411, a vertical screw 412, a mounting block 413, a vertical drive chain 414, and a first follower wheel 415. The output shaft of the vertical drive motor 411 coaxially mounted with a first gear, and the vertical drive chain is meshed with the first gear and the first driven wheel, respectively. The manipulator mechanism is socketed to the body of the vertical screw 412 and is fixedly connected with the chain of the vertical drive chain.

In some embodiments, the two-dimensional linear slide mechanism is connected with a vertical drive assembly, for driving the vertical drive assembly to move in a horizontal and transverse direction and a horizontal longitudinal direction. Specifically, the two-dimensional linear slide mechanism includes a transverse drive motor 421, a transverse screw, a mounting plate 423, a transverse rack belt 424, a longitudinal drive motor 431, a longitudinal drive chain 432, a second follower wheel 433, and a guiding strip 434. The transverse screw and the transverse rack belt 424 are fixed to the mounting plate 423, respectively, the mounting block 413 is socketed to the body of the transverse screw, the transverse drive motor 421 is fixed to the mounting plate 423, a second gear is coaxially mounted on the output shaft of the transverse drive motor 421, and the second gear is meshed with the transverse rack belt 424. The longitudinal drive motor 431, the guiding strip 434, and the second follower wheel 433 are fixed to the mounting frame 320, a third gear is coaxially mounted on the output shaft of the longitudinal drive motor 431, and the longitudinal drive chain 432 is separately connected in engagement with the third gear and the second driven wheel 433. The guiding strip 434 is calipered on the mounting plate 423, and the mounting plate 423 is connected to the longitudinal drive chain 432.

In some embodiments, the biological sample rack 210 includes a first rack body 810 and a second rack body 820. The first rack body 810 is symmetrically disposed on two opposite inner walls within the cabinet body 100, and the second rack body 820 is disposed between the first rack body 810 and parallel to the first rack body 810. In some embodiments, a count of the second rack body 820 may be 1, or at least 2 back-to-back bi-directional rack bodies each parallel to the first rack body 810. The sampling travel channel includes a first travel channel 830 disposed between the first rack body 810 and the second rack body 820, or between two adjacent second rack bodies 820, and a second travel channel 840 that conducts each first travel channel 830. The second traveling channel 840 may be available for the vertical screw 412 to realize a span between adjacent first traveling channels 830. The storage capacity of the cabinet body 100 may be substantially increased by employing this technical solution.

In some embodiments, a clearance channel 850 is also provided on the second rack body 820. In some embodiments, the height of the clearance channel 850 is no less than the height of the manipulator mechanism 500. Specifically, the clearance channel 850 is provided on the second rack body 820 near the side of the second travel channel 840 in this example. By adopting this technical solution, it is possible to enable the manipulator mechanism 500 to span between the adjacent first travel channel 830, which in turn reduces the design width of the second travel channel 840. In this example, the clearance channel 850 is provided at the top of the second rack body 820. In some embodiments, the clearance channel may also be designed at the waist, bottom, etc., of the second rack body 820.

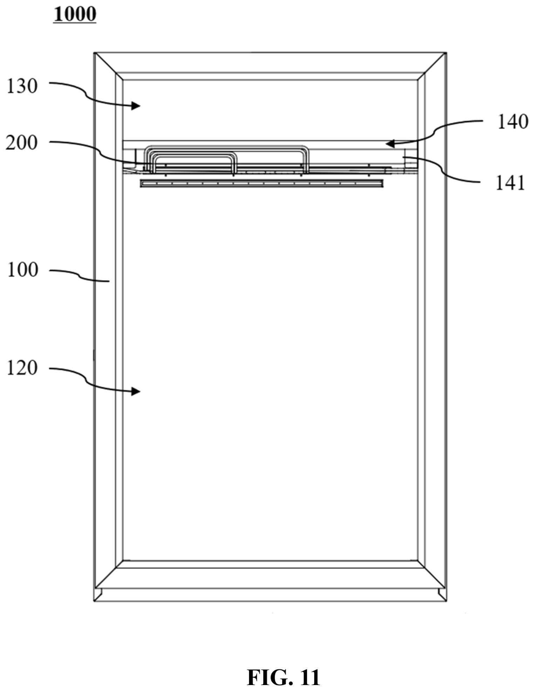

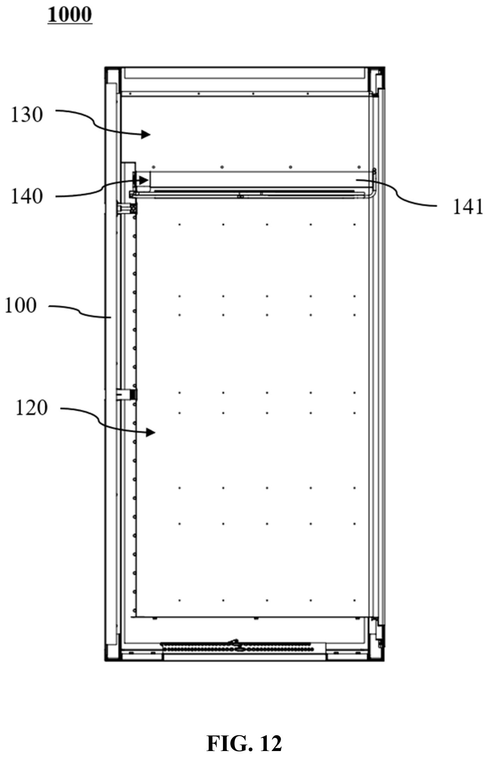

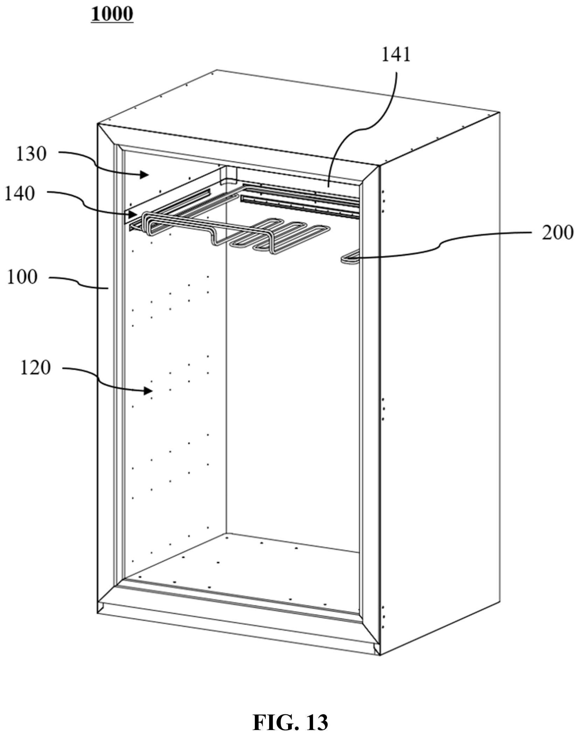

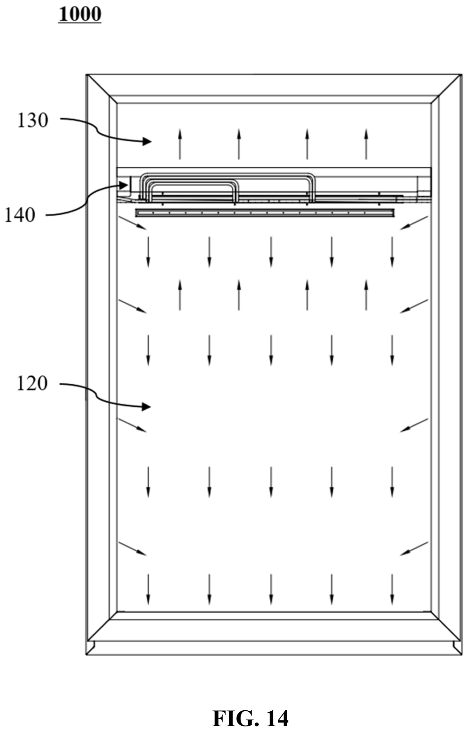

Some embodiments of the present disclosure provide a low-temperature preservation cabinet 1000 for a biological sample (or a refrigerated freezer device for storing the biological sample). FIG. 11 is a front view of a low-temperature preservation cabinet for a biological sample according to some embodiments of the present disclosure. FIG. 12 is a side view of a low-temperature preservation cabinet for a biological sample according to some embodiments of the present disclosure. FIG. 13 is a three-dimensional view of a low-temperature preservation cabinet for a biological sample according to some embodiments of the present disclosure. FIG. 14 is a schematic diagram illustrating gas flow in a low-temperature preservation cabinet for a biological sample according to some embodiments of the present disclosure. Devices such as the door body are hidden from view in the figure, where the lines with arrows up in FIG. 14 indicate the flow direction of hot air and the lines with arrows down indicate the flow direction of cold air.

In some embodiments, the preservation cabinet 1000 includes a cabinet body 100 (also referred to as a case), and an evaporator 200. The cabinet body 100 defines an accommodating cavity, the accommodating cavity being divided into a storage region 120, an equipment region 130, and a temperature isolation region 140 provided between the equipment region 130 and the storage region 120. The storage region 120 is configured to store the biological sample, and the storage region 120 is also configured to place a manipulator mechanism (also referred to as a pick up-and-place device), the equipment region 130 is configured to place a driving device, the driving device is configured to drive the manipulator mechanism for picking up and placing the biological sample, and the temperature isolation region 140 is provided with a temperature isolation device 141. The evaporator 200 is provided in the cabinet body 100 in the region corresponding to the storage region 120 (which may be partially or completely, for example, the sides, back, and top of the storage region 120 are designed with evaporation pipelines to provide the storage region 120 with the desired temperature) and the temperature isolation region 140 to, together with the temperature isolation device 141, make the temperature of the storage region 120 lower than the temperature of the equipment region 130.

The preservation cabinet 1000 makes the temperature of the storage region 120 lower than the temperature of the equipment region 130 by setting the temperature isolation device 141 and the evaporator 200 so that the driving device may be in a relatively high temperature. This reduces the cost by eliminating the need to use a driving device that is effective in resisting low temperatures. In some embodiments, in addition to providing the evaporator 200 in the region corresponding to the cabinet body 100 and the storage region 120, the evaporator 200 may also be provided in the temperature isolation region 140, thereby making the temperature of the storage region 120 more uniform, ensuring the storage effect of the biological sample.

The placement of the internal driving device for refrigeration and freezing devices that store biological samples, such as automated refrigerators and cold storage facilities is a tough problem. If the driving device is placed directly into the refrigerated freezer device, because of its internal ultra-low temperature (such as -80 °C) is far below the operating environment of the motor and other driving device, the use of low-temperature-resistant special components in this case, the price of such components is usually much higher, which directly affects the competitiveness of the whole machine, in addition to the ultra-low temperature environment for the normal operation of the driving device has a lot of risks that are difficult to estimate.

Some embodiments of the present disclosure have a new design for the cabinet body 100, with the cabinet body 100 separated into two open spaces. The upper space operates at a temperature of about -40 °C (merely by way of example, which may be determined according to the actual situation), and the lower space operates at a temperature of about -80 °C (merely by way of example, which may be determined according to the actual situation). The driving device is placed right in the upper region with a higher temperature, which is also relatively friendly for the operation of the driving device, and also provides for the reliability of the operation of the complete mechanism.

In some embodiments, the temperature isolation device 141 includes a divider member for connecting a portion of the cabinet body 100 forming the storage region 120 (which may be a liner of the storage region 120), and, a portion of the cabinet body 100 forming the equipment region 130 (which may be a liner of the equipment region 130, an outer shell may be disposed outside of each of the liner, and an opening may be formed in the front of the outer shell between the outer shell and each of the liner, and the preservation cabinet 1000 may include an opening frame provided in the opening) to reduce heat transfer between the portion of the cabinet body 100 forming the storage region 120, and, the portion of the cabinet body 100 forming the equipment region 130 (which may be wrapped around an outer side of the liner of the storage region 120).

In some embodiments, the portion of the cabinet body 100 forming the storage region 120, and, the portion of the cabinet body 100 forming the equipment region 130 may be made of sheet metal, and the divider member may be plastic (e.g., a circle of plastic pieces), and in other embodiments, the divider member may also be made of other materials.

In some embodiments, the divider member includes at least a first divider portion that extends along a first direction, a second divider portion that extends along a second direction that is different from the first direction, and a connection portion. The connection portion is configured to connect the first divider portion and the second divider portion. As a result, such a divider member may be adapted to a complex shape of the accommodating cavity.

In some embodiments, the connection portion is provided with one of an insertion projection and an insertion slot, and the first divider portion and the second divider portion are provided with the other of the insertion projection and the insertion slot. As a result, the stability of the connection between the connection portion and the first divider portion and the second divider portion is improved.

In some embodiments, the preservation cabinet 1000 further includes at least one mounting box 900, each of the mounting boxes 900 being mounted in the temperature isolation region 140, and each of the mounting boxes is provided with at least a portion of the evaporator 200. The mounting box is provided with an opening on a side near the storage region 120, and the mounting box is provided with a closed structure on a side near the equipment region 130. As a result, more of the cold volume enters the storage region 120, which facilitates the storage of the biological sample and avoids interfering with the driving device.

In some embodiments, at least a portion of the evaporator 200 disposed inside the mounting box is disposed on a side of the mounting box close to the storage region 120. The temperature isolation device 141 includes a temperature isolation member disposed inside a side of the mounting box close to the equipment region 130 to reduce the transfer of cold generated by the at least a portion of the evaporator 200 disposed inside the mounting box to the equipment region 130. Specifically, the temperature isolation member may be made of a material such as foam, or the like. Specifically, the temperature isolation member may be made of rubber, plastic, or the like. Therefore, it is further ensured that the cold volume enters more into the storage region 120, which facilitates the storage of the biological sample and avoids interference with the driving device.

In some embodiments, there are a plurality of mounting boxes spaced apart, which not only ensures that more of the cold volume enters the storage region 120 but also provides space for the manipulator mechanism to move.

In some embodiments, the preservation cabinet 1000 may also include a compressor, provided on a first side of the cabinet body 100 and located outside the accommodating cavity, for forming a refrigeration system together with the evaporator 200 (it is to be understood that the entire refrigeration system may also include other devices such as a throttling device, a condenser, or the like, and the refrigeration system of the present embodiment may be a dual-engine duplexed refrigeration system, and in the present embodiment, there may be two sets of dual-engine duplexed refrigeration systems, and one of the dual-engine duplexed refrigeration systems may be used as a backup refrigeration system). The plurality of mounting boxes includes a first mounting box provided in a region of the temperature isolation region 140 that corresponds to the first side, a second mounting box provided in a region of the temperature isolation region 140 that corresponds to the second side opposite to the first side. The evaporator 200 is configured to enter the second mounting box first and then enter the first mounting box before being connected to the compressor. This avoids the complex winding of the return line for the connection between the evaporator 200 and the compressor.

In some embodiments, the plurality of mounting boxes may further include a third mounting box, the third mounting box being disposed between the first mounting box and the second mounting box, and the third mounting box being connected to the first mounting box by a connection member to allow the evaporator 200 to first enter the second mounting box, and enter the first mounting box and then into the third mounting box through the connection member, then through the connection member to the first mounting box, then enter the first mounting box through the connection member and then to the compressor. The third mounting box is spaced apart from the second mounting box. Therefore, the manipulator mechanism may be installed through the gap between the third mounting box and the second mounting box, which is easy to install and improves the installation efficiency.

In some embodiments, the equipment region 130 is located above the storage region 120, whereby the cold air settling reduces the impact on the temperature of the mechanism placement region (i.e., the equipment region), and the air inside the box naturally circulates when working, with the cold air settling and the hot air rising, dividing the top and bottom into two temperature spaces, bounded by the temperature isolation zone 140. The present embodiment cuts off the heat conduction between the sheet metal of the storage region and the sheet metal of the mechanism region by designing a specialized temperature isolation region 140, and then the refrigeration pipelines are all arranged in the storage region 120 such that the air inside the box in the case of natural convection creates two temperature zones namely the -40°C zone in the upper portion and -80 °C zone in the lower portion.

The preservation cabinet 1000 shown in some embodiments of the present disclosure simplifies the structure of the entire cabinet body 100, improves the reliability, and avoids potential risks. In addition to increasing the efficiency of the assembly of the cabinet body 100, the production costs may be reduced accordingly. In some embodiments, the accommodating cavity may have a forward-facing opening, avoiding the problem of poor airtightness due to the opening above. In addition, some embodiments of the present disclosure are divided into two spaces inside the complete cabinet body 100, with the sealing of the entire cabinet body 100 not broken.

In order to improve the level of automation of the biological sample storage device, some automation devices are provided in the accommodating cavity of the biological sample storage device. However, this type of biological sample storage device is prone to leakage of cold, which is unfavorable to the storage of the biological sample.

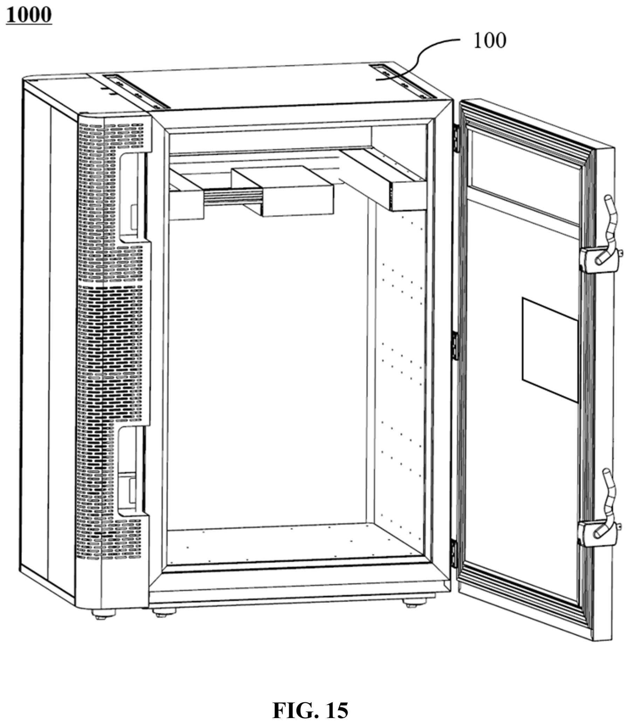

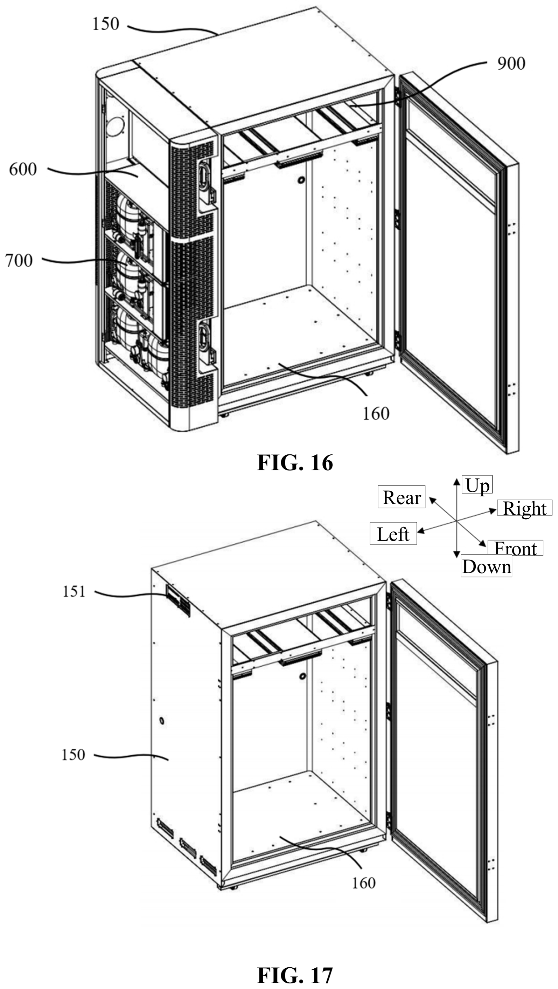

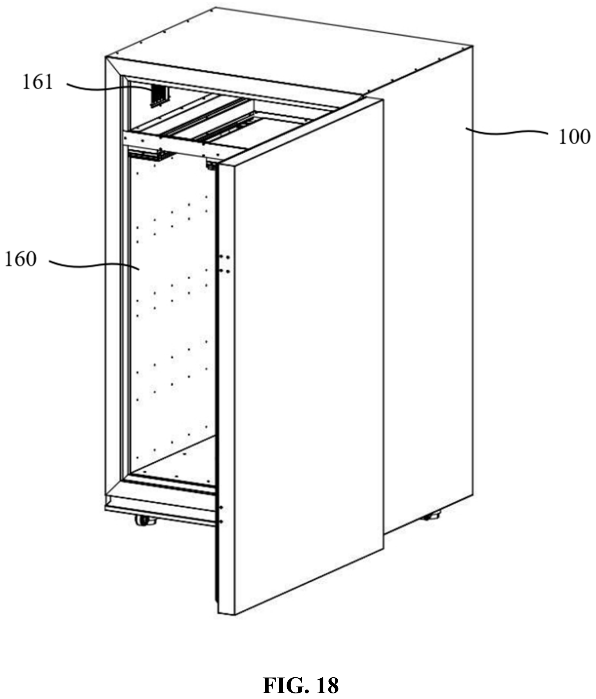

Some embodiments of the present disclosure provide a low-temperature preservation cabinet 1000 for a biological sample (also referred to as a biological sample storage device), FIG. 15 is a schematic diagram illustrating a structure of a low-temperature preservation cabinet for a biological sample without a drive system and shelves according to some embodiments of the present disclosure. FIG. 16 is a schematic diagram illustrating the structure of the low-temperature preservation cabinet for the biological sample shown in FIG. 15 without a side panel of a cabinet body. FIG. 17 is a schematic diagram illustrating the structure of the low-temperature preservation cabinet for the biological sample shown in FIG. 16 without a side cabinet. FIG. 18 is a schematic diagram illustrating another view of the structure shown in FIG. 17.

The low-temperature preservation cabinet 1000 for a biological sample includes a cabinet body 100 including an outer shell 150 defining a shell cavity, and an inner liner 160 provided in the shell cavity, the inner liner 160 defining an accommodating cavity. In some embodiments, the outer shell is provided with a first wiring port, the inner liner is provided with a second wiring port, and the first wiring port and the second wiring port have different positions in at least two directions. In some embodiments, the outer shell 150 is provided with a first wiring port for placing a first wiring box 151, the inner liner 160 is provided with a second wiring port for placing a second wiring box 161, and the first wiring box 151 and the second wiring box 161 have different positions in at least two directions. In some embodiments, a storage region and a drive system are provided in the accommodating cavity. The drive system is connected to a manipulator mechanism to drive the manipulator mechanism to move to pick up and place a biological sample stored in the storage region, the drive system is connected to one end of a cable, and the other end of the cable extends out of the shell cavity after passing through the second wiring box 161 and the first wiring box 151, and the cable is configured to realize power supply and/or control of the drive system. In some embodiments, the other end of the cable passes through the second wiring port via the second wiring box, and the other end of the cable passes through the first wiring port via the first wiring box.

In some embodiments, the first wiring port (or the first wiring box 151) and the second wiring port (or the second wiring box 161) of the preservation cabinet 1000 have different positions in at least two directions, i.e., in at least two of the transverse direction (or called left-right direction), the up-down direction, and the front-rear direction of the cabinet body 100. For example, the first wiring box 151 and the second wiring box 161 have different positions in the transverse direction and the up-down direction; the first wiring box 151 and the second wiring box 161 have different positions in the transverse direction and the front-rear direction, the first wiring box 151 and the second wiring box 161 have different positions in the front-rear direction and the up-down direction, or the first wiring box 151 and the second wiring box 161 have different positions in the transverse direction, the front-rear direction, and the up-down direction. Therefore, the cold volume of the biological sample storage device is more difficult to leak through the first wiring box 151 and the second wiring box 161, which facilitates the storage of the biological sample.

In some embodiments, a side cabinet may be provided on one side of the cabinet body 100, and a control device, a compressor 700, a condenser, or the like, may be provided in the side cabinet. The side cabinet may have a cabinet door. In some embodiments, a mounting mechanism 600 is fixed to the outer wall of the housing 150, the mounting mechanism 600 is mounted with a control device, and the control device is connected to the other end of the cable. The mounting mechanism 600 may be a mounting bracket, a mounting plate, or the like, and the control device may be an electrical control cabinet, or the like. Specifically, the mounting plate is fixed to the outer surface of the side wall of the housing 150, and the mounting plate is fixed to the electrical control cabinet, thereby avoiding the cold affecting the electrical control cabinet. In other embodiments, the other end of the cable may be connected to a device such as a power supply.

In some embodiments, the mounting mechanism 600 is provided below the compressor 700 that is fixed to the outer wall of the housing 150. That is to say, the position of the electronical control cabinet is higher, and since the compressor 700 may have water on its return end when it is in operation, there is a risk of water dripping down to the control mechanism affecting the safety of the circuits if the control mechanism is underneath. Furthermore, the refrigeration system such as the compressor 700 being placed underneath may result in a low center of gravity as a whole, and a higher degree of stability.

In some embodiments, an insulating device is provided between the outer shell 150 and the inner liner 160. The insulating device may be foamed, etc., to avoid cold leakage.

In some embodiments, a temperature isolation device is provided between the drive system and the storage region, and the preservation cabinet 1000 includes a refrigeration system, and the refrigeration system is configured to provide a cold volume to a corresponding region of the storage region. Therefore, the drive system may not be in a state where the temperature is too low, and the normal operation of the drive system is ensured. The drive system is located above the storage region, and the second wiring box 161 is located above the temperature isolation device. This approach reduces the effect of cold air on the drive system due to the principle that hot air naturally floats up and cold air naturally sinks. It may be appreciated that in other embodiments, the drive system may also be located in other locations in the storage region.

A material lifting device is a transfer device for moving items in and out of shelves and may be used in a biological sample storage cabinet, which stores the biological sample by refrigerating or freezing. At present, items are moved in and out of two sets of adjacent shelves by a manipulator between two sets of adjacent shelves for removing and placing the material. Related technology in the two sets of adjacent shelves in the transfer of materials using bi-directional telescopic manipulator, the defect is that purely can only be realized in two directions of the switching and cannot be achieved in the other direction of adjustment. In response to the problems of the prior art, embodiments of the present disclosure provide a material lifting device (or referred to as a manipulator mechanism) to solve at least one of the above technical problems.

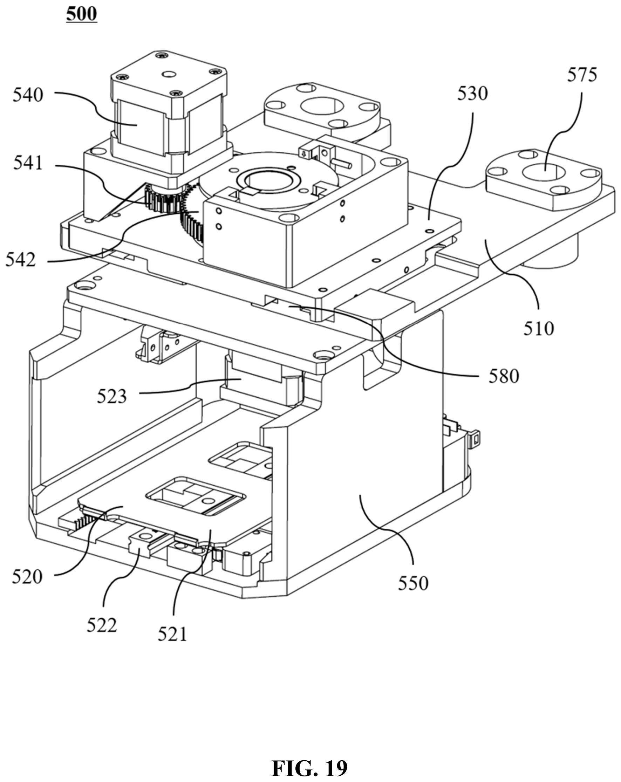

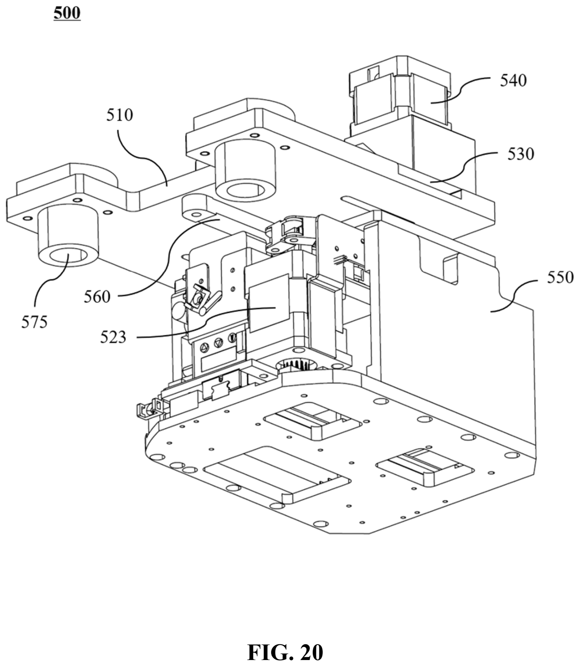

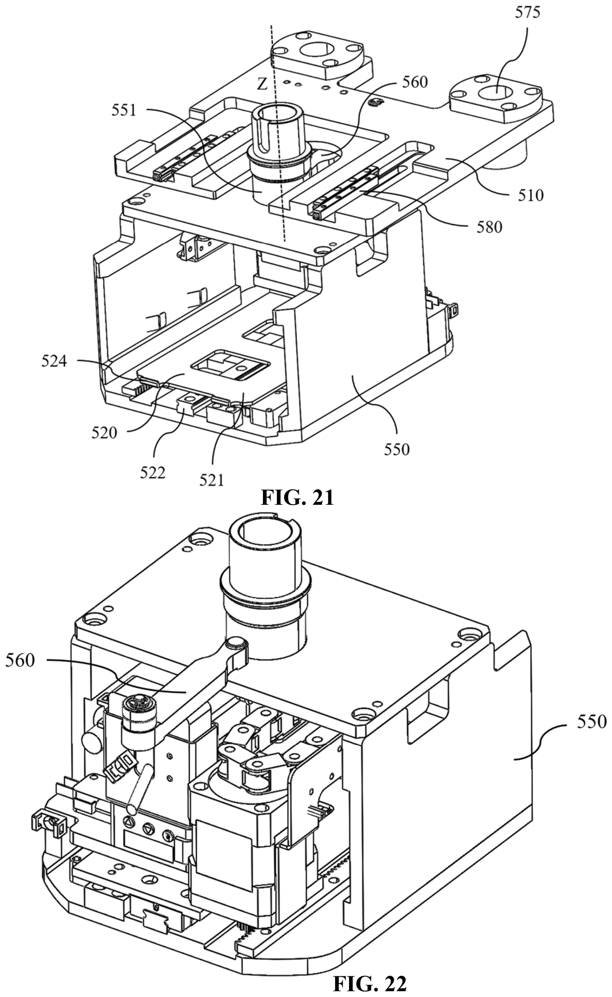

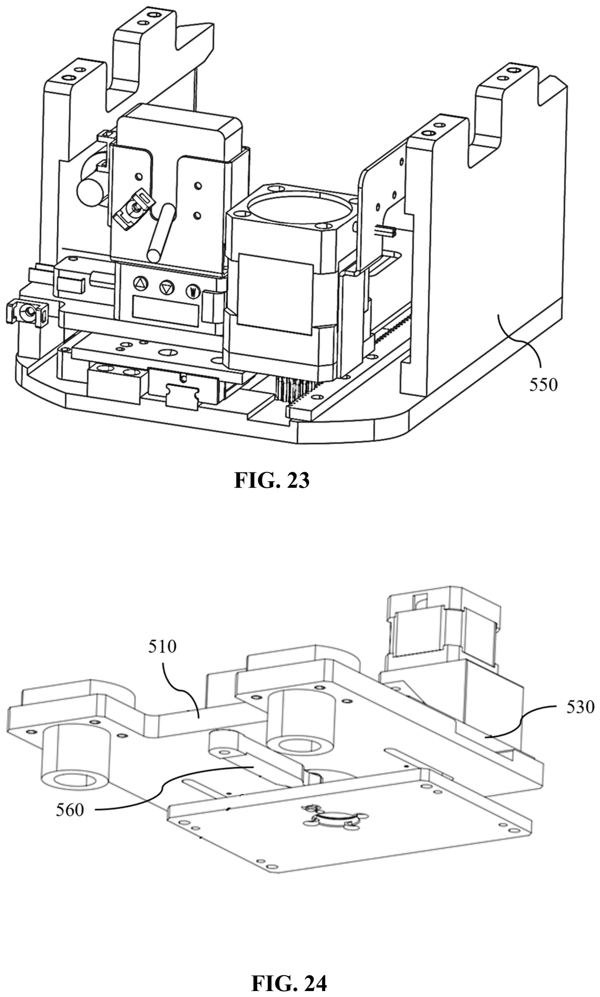

FIG. 19 is a schematic diagram illustrating a first view of a manipulator mechanism according to some embodiments of the present disclosure. FIG. 20 is a schematic diagram illustrating a second view of a manipulator mechanism according to some embodiments of the present disclosure. FIG. 21 is a schematic diagram illustrating a first partial structure of a manipulator mechanism according to some embodiments of the present disclosure. FIG. 22 is a schematic diagram illustrating a second partial structure of a manipulator mechanism according to some embodiments of the present disclosure. FIG. 23 is a schematic diagram illustrating a third partial structure of a manipulator mechanism according to some embodiments of the present disclosure. FIG. 24 is a schematic diagram illustrating a fourth partial structure of a manipulator mechanism according to some embodiments of the present disclosure.

In some embodiments, the manipulator mechanism 500 includes a pick-up arm 520 and a steering mechanism. The pick-up arm 520 is configured to pick up and place the biological sample. The steering mechanism includes a rotating rack 550, a manipulator motor 540, an offset guiding mechanism, and a base 530. The pick-up arm 520 is mounted on the rotating rack 550, and the manipulator motor 540 is mounted on the base 530. The offset guiding mechanism includes a mounting base 510 and a connecting rod 560. The mounting base 510 is disposed between the base 530 and the rotating rack 550, and the mounting base 510 is slidably connected with the base 530 in a first linear direction. One end of the connecting rod 560 is hinged to the base 530 and the other end of the connecting rod 560 is hinged to the rotating rack 550. As the rotating rack 550 rotates, the connecting rod 560 drives the base 530 to slide relative to the mounting base 510 in the first linear direction. In some embodiments, the present solution enables the rotating rack 550 to achieve a synchronous sliding offset of the rotating center axis (as shown by the Z-axis shown in FIG. 21) during rotation by having an additional rotating center offset mechanism. Thus, the protruding length of the rotating rack 550 may be adjusted in different rotational positions. In some embodiments, the mounting base 510 may be a mounting block 413.

In some embodiments, referring to FIG. 19 to FIG. 23, the manipulator mechanism may be a material lifting device, the material lifting device including a pick-up arm 520 for picking up and placing the material and a steering mechanism. The steering mechanism includes a base 530, a manipulator motor 540, and a rotating rack 550. The manipulator motor 540 is mounted on the base 530, and the manipulator motor 540 is configured to drive the rotating rack 550 to rotate. The rotating rack 550 is mounted with the pick-up arm 520. The material lifting device also includes a rotating center offset mechanism, the rotating center offset mechanism is configured to drive the rotating center axis of the rotating rack 550 to translate along the first linear direction when the rotating rack 550 is in motion, and the manipulator motor 540 is also configured to drive the rotating center offset mechanism to move. The center axis direction of the rotating rack 550 is mutually perpendicular to the first linear direction. In some embodiments, the rotating center offset mechanism is capable of causing the rotating rack 550 to rotate with a translational movement of the rotating center axis of the rotating rack 550 with respect to the mounting base 510. In some embodiments, the center axis direction of the rotating rack 550 may be a vertical direction (or an up and down direction).

Some embodiments of the present disclosure optimize the material lifting device so that the manipulator motor 540 may drive the rotating rack 550 to be adjusted in a plurality of directions. At the same time, by adding a rotating center offset mechanism, it is convenient to realize that the rotation process of the rotating rack 550 is not in situ, and the rotating center axis of the rotating rack 550 generates a sliding offset during the rotation process, so that the protruding length of the rotating rack 550 may be adjusted in different rotation positions. In some embodiments, the length of the rotating rack 550 extending along the length direction (the horizontal direction perpendicular to the arrangement direction of the adjacent shelves) of a shelf gap is greater than the length of the rotating rack 550 extending in a direction adjacent to the shelf (the arrangement direction of the adjacent shelves) to satisfy the normal rotation of the rotating rack 550 in a narrow channel, to effectively control the occupying space required for the rotation of the rotating rack 550 and to ensure the steering of the material lifting device.

In some embodiments, the pick-up arm 520 includes a sliding member 521 for picking up and placing the material, the sliding member 521 being slidably connected to the rotating rack 550 via guide rail 522. The pick-up arm 520 further includes a pick-up drive mechanism 523 for driving the sliding member 521 along the guide rail 522 in a straight line. In some embodiments, the pick-up drive mechanism 523 includes a motor, the power output of the motor being connected to a gear, the gear being meshed with a straight rack mounted to the rotating rack 550. The motor may be mounted to the sliding member 521.

In some embodiments, for the sliding of the sliding member 521, a slider is provided at the bottom of the sliding member, and the rotating rack 550 is provided with a slide rail that is slidingly connected to the slider. Alternatively, the bottom of the sliding member is provided with rollers. The rotating rack 550 is provided with a slide rail for guiding the sliding direction of the rollers. In some embodiments, the sliding member 521 may be a shovel plate (as shown in FIG. 19), a fork arm, or a clamping mechanism, etc. When the sliding member is a shovel plate, the end portion of the shovel plate slidingly extending out of the rotating rack 550 is provided with an upwardly-extending limiting protrusion 524. The shovel plate may slide out of the rotating rack by moving underneath the material and then moving upwardly to lift the material, and then the shovel plate slides to retract back into the rotating rack with the material. When the sliding member is a fork arm, the material is provided with a fork hole for insertion into the fork arm. The fork arm slides to extend out of the rotating rack and extends into the fork hole and then moves upward, driving the material upward, and then the fork arm drives the material to slide and retract back to the rotating rack. When the sliding member is a clamping mechanism, the fork arm slides out of the rotating rack, and when it is located on two sides of the material, the clamping mechanism grips the material, moves upward, and drives the material to rise up, and then, the fork arm drives the material to slide back to the rotating rack.

In some embodiments, the steering mechanism further includes a mounting base 510, the mounting base 510 being configured to support the steering mechanism. One side of the mounting base 510 is provided with a guiding hole 575 for slidingly connecting a guiding post. At least two guiding holes may be provided. The at least two guiding holes may be arranged along a second linear direction perpendicular to the first linear direction. When the rotating center axis translate along the first linear direction, the rotating center axis moves from the side close to the guiding hole 575 to the side away from the guiding hole 575, or the rotating center axis moves from the side away from the guiding holes 575 to the side close to the guiding hole 575. With the rotating center offset mechanism, the rotating rack 550 may avoid the guiding post during rotation. The guiding post may be a post whose guiding direction is in a vertical direction. The guiding post is configured to guide the mounting base 510 up and down. In some embodiments, the guiding post may be a vertical screw 412.

In some embodiments, the manipulator motor 540 is disposed on either side of the rotating rack 550 in a center axis direction of the guiding hole, controlling the overall occupying space of the device and controlling the size of the occupying channel. Specifically, the longitudinal axis direction of the guiding hole may be a vertical direction, the manipulator motor 540 is disposed on top of the base 530, and a power output shaft of the manipulator motor 540 is disposed vertically to facilitate control of the occupying space.

In some embodiments, the power output shaft of the manipulator motor 540 is connected to a gear reduction mechanism, and a power output end of the gear reduction mechanism is connected to the rotating rack 550. The gear reduction mechanism includes an active gear 541 mounted on the power output shaft of the manipulator motor 540 and a driven gear 542, and the active gear 541 meshes with the driven gear 542. The driven gear 542 is mounted on a rotating shaft 551 of the rotating rack 550, and the rotating shaft 551 of the rotating rack 550 is rotationally connected to the base 530 via bearing.

In some embodiments, the rotating center offset mechanism includes a guiding assembly 580 for guiding the base 530 to be slidably connected to the mounting base 510 in the first linear direction. The guiding assembly 580 may include mutually matching slide rails and slider blocks. Alternatively, the guiding assembly includes a slide rail and a rolling wheel that travels along the slide rail. In some embodiments, one end of the connecting rod 560 is hinged to the mounting base 510, and the other end of the connecting rod 560 is hinged to the rotating rack 550. The hinge may be understood as a rotational hinge. During rotation of the rotating rack 550, the connecting rod 560 drives the rotating rack 550 and the base 530 to slide in the first linear direction with respect to the mounting base 510, realizing a translational movement of the rotating center axis. The base 530 is rotatably connected to the rotating rack 550, and the base 530 slides with the rotating rack 550 along the first linear direction with respect to the mounting base 510. In some embodiments, a direction of a line connecting a rotating center axis of a hinge linkage between the rotating rack and the rotating center axis of the rotating rack is parallel to the first linear direction. In some embodiments, the first linear direction may be a guiding direction (or a length direction) of the guiding assembly 580. In some embodiments, a side of the rotating rack 550 close to the guiding hole is hinged to the connecting rod 560. The connecting rod 560 is hinged to the side of the mounting base 510 close the guiding hole. In some embodiments, the axial length of the connecting rod is greater than a distance between the rotating center axis of the hinge linkage of the connecting rod with the rotating rack 550 and the rotating center axis of the rotating rack 550.

In some embodiments, during circumferential rotation of the rotating rack 550 at 180°, the base 530 slides from the side close to the guiding hole to the side away from the guiding hole as the circumferential direction is rotated from 0° to 90°. The base 530 slides from the side away from the guiding hole to the side close to the guiding hole as the circumferential direction is rotated from 90° to 180°. FIG. 19 and FIG. 20 show schematic diagrams of the structure of the rotating rack 550 in a 90° position state. When the rotating rack 550 is rotated to 0° and 180°, it is possible to switch the picking up and placing of materials on the adjacent two sets of shelves, respectively.

In some embodiments, the mounting base 510 may be mounted on a lifting slide of a three-axis linear module when the present device (material lifting device) is used, which facilitates the three-dimensional movement of the device as a whole.

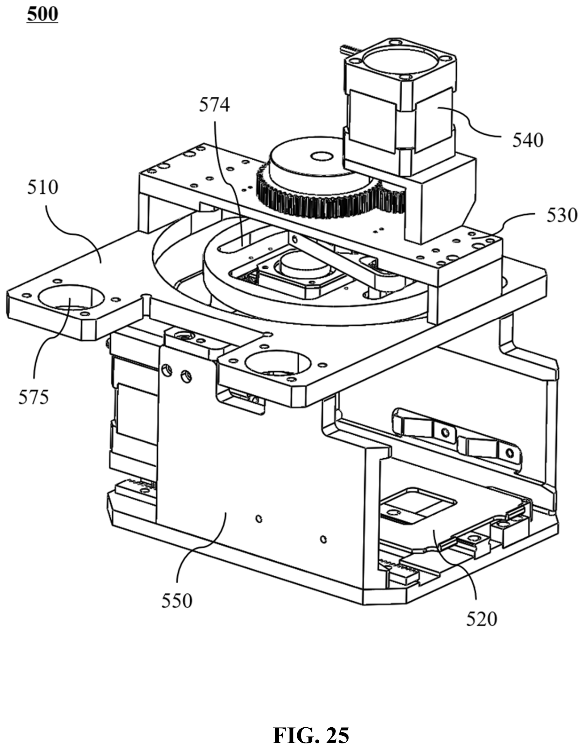

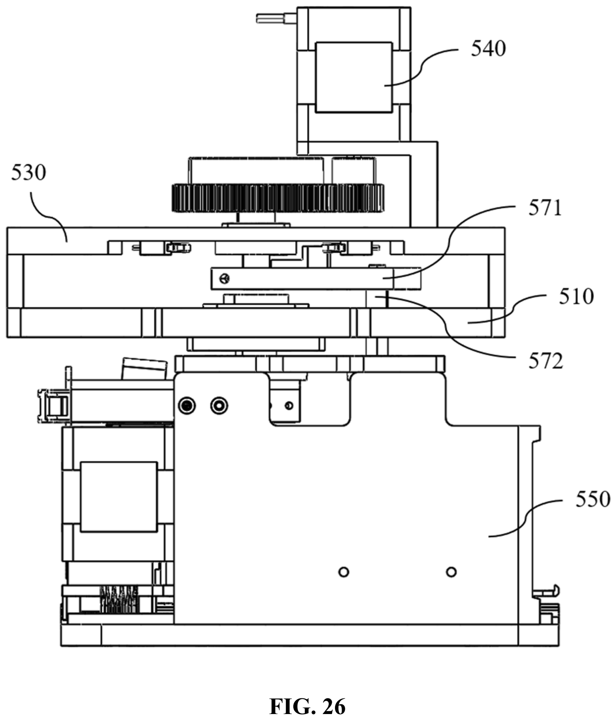

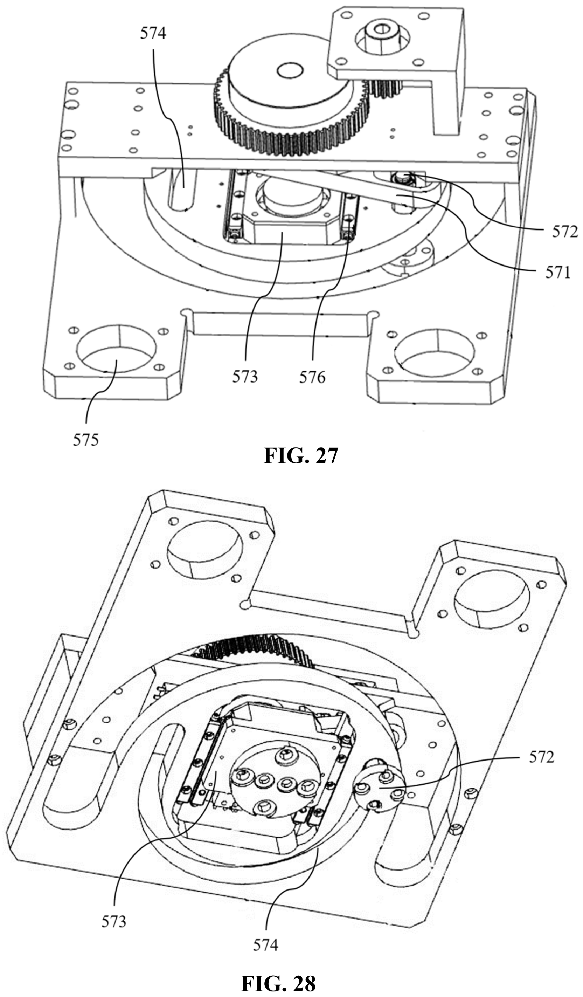

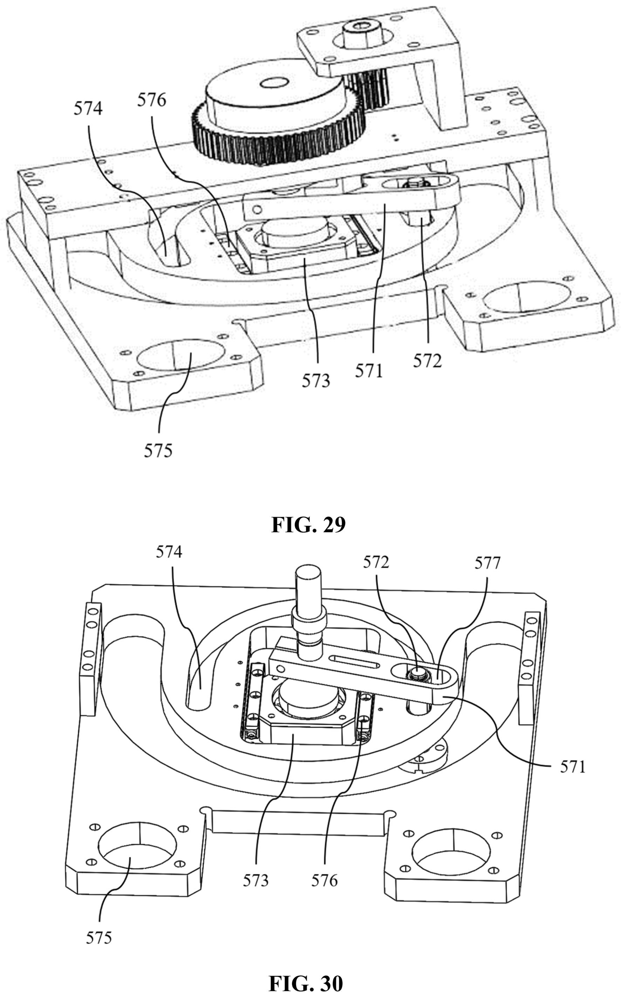

FIG. 25 is a schematic diagram illustrating a structure of a manipulator mechanism according to another embodiment of the present disclosure. FIG. 26 is a schematic diagram illustrating a structure of another view of a manipulator mechanism according to another embodiment of the present disclosure. FIG. 27 is a schematic diagram illustrating a first view of a partial structure of a manipulator mechanism according to another embodiment of the present disclosure. FIG. 28 is schematic diagram illustrating a second view of a partial structure of a manipulator mechanism according to another embodiment of the present disclosure. FIG. 29 is schematic diagram illustrating a third view of a partial structure of a manipulator mechanism according to another embodiment of the present disclosure. FIG. 30 is a schematic diagram illustrating another partial structure of a manipulator mechanism according to another embodiment of the present disclosure.

In some embodiments, referring to FIG. 25 to FIG. 30, the rotating center offset mechanism of the manipulator mechanism 500 may include a guiding assembly for guiding the rotating center axis of the rotating rack 550 to slide in the first linear direction. The guiding assembly includes a slide rail 576 and a slide block 573. A central part of the slide block 573 is rotationally connected to the rotating rack 550 via rotating axis. A power output end of the manipulator motor 540 is connected to an end of an oscillating arm 571 via gear mechanism, and the other end of the oscillating arm 571 is movably connected to a guiding rod 572. The guiding rod 572 is connected to the rotating rack 550. The base 530 and the slide rail 576 are mounted on the mounting base 510, and the mounting base 510 is provided with a guiding groove 574 for guiding the movement of the guiding rod 572, and the guiding groove 574 is U-shaped.

In some embodiments, the guiding hole 575 for slidingly connecting the guiding post is provided on one side of the mounting base 510. In some embodiments, the guiding hole 575 and an open end of the guiding groove 574 are provided on different sides of the mounting base 510. For example, when the guiding hole is disposed on the left side of the mounting base, the open end of the guiding groove is disposed on the right side of the mounting base, and the open end of the guiding groove is oriented to the right. In some embodiments, the guiding hole 575 and the guiding groove 574 are provided on different sides of the mounting base 510. In some embodiments, the guiding groove 574 has an opening direction (e.g., an opening direction of a U-shaped guiding groove) toward the guiding hole 575.

In some embodiments, the oscillating arm 571 is movably connected with a guiding rod 572 at the other end. The oscillating arm 571 may be provided with an elongated guiding hole 577. The length direction of the guiding hole 577 is parallel to the length direction of the oscillating arm 571. The end portion of the guiding rod 572 is rotatably and slidably mounted within the guiding hole 577. For example, the end portion of the guiding rod 572 is threaded with a nut. The guiding hole 577 includes an upper portion and a lower portion disposed above and below, with the upper portion being a nut-activated groove, and the lower portion being a sliding groove for the guiding rod 572. A projected region of the inner contour of the upper portion is disposed peripherally to a projected region of the inner contour of the lower portion.

In some embodiments, the mounting base 510 is provided with an oscillating arm 571 and a rotating rack 550 on different sides along the direction of the rotating center axis. The center axis direction of the rotating axis is the direction of the rotating center axis of the rotating rack 550.

In some embodiments, the base 530 is mounted to the mounting base 510, which may be an integrated base-mount structure. Alternatively, the base is removably connected to the mounting base. Alternatively, the base and the mounting base are welded and fixedly connected, etc.

In some embodiments, the guiding groove 574 includes two linear ends (a first linear end and a second linear end, respectively) disposed side by side, the two linear ends being connected by an arcuate end. During the swinging process of the oscillating arm 571 driven by the manipulator motor 540, the rotating rack 550 is driven to slide outward along the first linear end of the guiding groove 574 along the first linear direction (the rotating rack 550 slides from the side close to the guiding hole 575 to the side away from the guiding hole 575, which means that the rotating center axis moves from the side close to the guiding hole 575 to the side away from the guiding hole 575), and the rotating rack 550 is driven to rotate along the arcuate end of the guiding groove (to achieve 180° rotation of the rotating rack),. Finally, the rotating rack 550 is driven to be retracted inwardly along the second linear end of the guiding groove 574 (the rotating rack slides from the side away from the guiding hole 575 to the side close to the guiding hole 575, i.e., the rotating center axis moves from the side away from the guiding hole 575 to the side close to the guiding hole 575). The oscillating arm 571 is provided with a guiding hole 577 for the movement of the guiding rod 572, and the length direction of the guiding hole is parallel to the length direction of the oscillating arm 571.

In some embodiments, FIGs. 25- 30 show schematic diagrams of the structure of the rotating rack in the 0° position state. At this point, the guiding rod 572 is located at the end of the guiding groove 574. When the rotating rack 550 is rotated to 0° and to 180°, the switch of picking up and placing of materials on the two adjacent sets of shelves may be realized respectively.

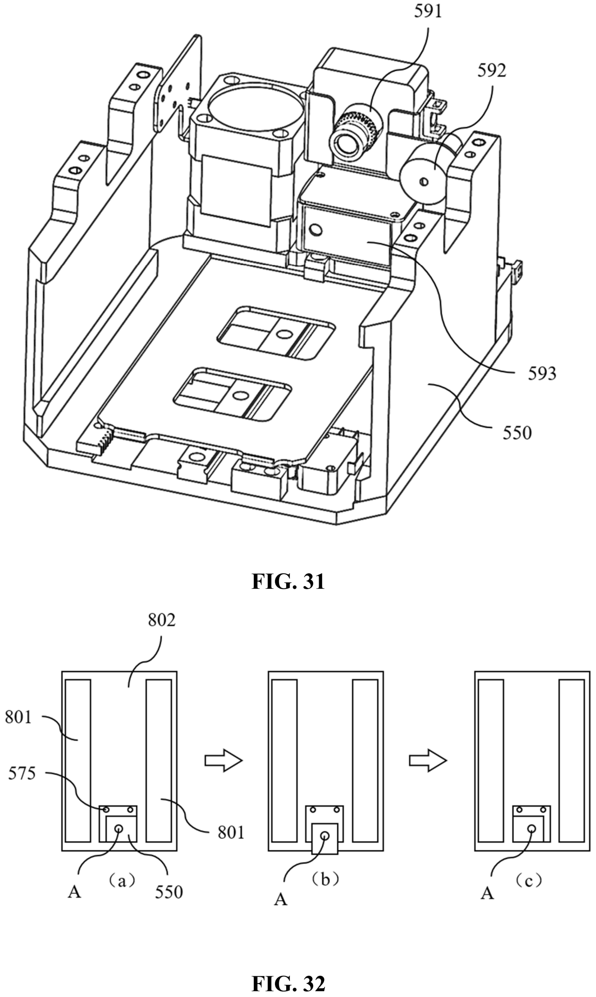

FIG. 31 is a schematic diagram illustrating a partial structure of a manipulator mechanism according to some embodiments of the present disclosure. In some embodiments, referring to FIG. 31, the rotating rack 550 is mounted with a camera device 591 and a light source 592 provided adjacent thereto. In some embodiments, the rotating rack 550 is also mounted with a laser range finder 593 for detecting the retracting and expanding process of the material driven by the pick-up arm.

In some embodiments, at least one side of a shelf in a width direction is provided with a passageway for the material lifting device to move, and a length direction of the passageway is parallel to the first linear direction. The length direction of the passageway is parallel to the length direction of the shelf, i.e., the direction in which the material lifting device may move in a straight line. The length direction of the passageway is perpendicular to the width direction of the shelf, and the width direction of the shelf is the direction in which the material on the shelf is to be picked up and placed (e.g., the width direction of the shelf is the left-right direction). The left or right side of the shelf may be used for picking up and placing the material. The length of the passageway is oriented in the front- back direction. The material lifting device may slide back and forth to pick up and place the material in different front and back positions of the shelf.

In some embodiments, the laser range finder 593 may be configured to detect if the shelf is deformed. In some embodiments, the manipulator mechanism may move sequentially along the shape of the shelf, and the laser range finder 593 may detect various parts of the shelf. By processing the various parts of the shelf measured by the laser range finder 593, it is possible to effectively determine whether the shelf is deformed.

FIG. 32 is a schematic diagram illustrating different states of circumferential rotation of a rotating rack of a manipulator mechanism according to some embodiments of the present disclosure. In some embodiments, referring to FIG. 32, a passageway 802 for the material lifting device to move is provided between two adjacent shelves 801, with the length direction of the passageway 802 being parallel to the first linear direction. The passageway 802 is directly opposite a shipping port (which may be opened and closed), which is located on the side of the mounting base away from the guiding hole 575. The shipping port is a window configured to conduct the outer side and the inner side of the cabinet body for picking up and placing the material.

In some embodiments, the rotating rack 550 adjusts the orientation of the pick-up arm as it rotates and the pick-up arm is oriented sequentially toward one of the two shelves 801, the shipping port, and the other of the two shelves 801. FIG. 32 (b) shows a schematic diagram of the rotating rack 550 of FIG. 32 after it has been rotated 90° circumferentially. FIG. 32 (c) shows a schematic diagram of rotating rack 550 of FIG. 32 (b) after it has been rotated 90° circumferentially.

In some embodiments, the rotating center axis of the rotating rack 550 is located on a side close to the guiding hole 575 when the pick-up arm is oriented toward either of the two shelves 801. Referring to FIG. 32 (a) and FIG. 32 (c), showing the pick-up arm toward two adjacent shelves 801, respectively.

In some embodiments, the rotating center axis of the rotating rack 550 is located on the side away from the guiding hole 575 when the pick-up arm is facing the shipping port, referring to FIG. 32 (b). The rotation of the rotating rack 550 enables switching of the pick-up arm toward the two shelves 801 or the shipping port. The rotating rack 550 is provided with a rotating center axis A, and the rotating center axis is located at A in FIG. 32.

In some embodiments, one end of the rotating rack 550 is an access end for the pick-up arm 520 to extend and retract. During rotation of the rotating rack 550, when the access end is facing the class A shelf or class B shelf, the rotating center axis of the rotating rack 550 slides to the side close to the guiding hole. When the access end faces the maintenance port (or shipping port) opened by the cabinet body, a rotating center baseline of the rotating rack 550 slides to the side away from the guiding hole.

In some embodiments, the cabinet body is opened with a maintenance port, the maintenance port facing the spacing channel. The maintenance port is rotationally connected with a flip door. When rotating rack 550 rotates and the pick-up arm 520 of the manipulator mechanism rotates from being oriented toward the class A shelf or the class B shelf to being oriented toward the maintenance port, the rotating rack 550 pushes the flip door outwardly. The maintenance port is a window for the outside of the cabinet body to conduct with the inside of the cabinet body for picking up and placing the material.

In some embodiments, with respect to the manner in which the flip door is rotated with the maintenance port, it may be that the top of the flip door is rotationally connected to the top of the maintenance port, facilitating the natural sagging of the flip door under gravity. Pushing the flip door to flip open during rotation of the rotating rack 550 allows the flip door to naturally sag closed when the rotating rack 550 is facing the shelf.

In some embodiments, the left or right end of the flip door is hinged to the maintenance port. A reset spring is provided at the hinged end of the flip door and the maintenance port. During switching of the rotating rack 550 from facing the shelves to facing the maintenance port, the flip door is pushed through the rotating rack 550 during rotation. When the flip door overcomes the spring force of the reset spring, the flip door flips open, and when the rotating rack 550 is facing the shelves, the flip door naturally drops down and closes.

The embodiments of the present disclosure may include but are not limited to the following beneficial effects. First, the temperature zoning in the cabinet body is realized, setting the biological sample in the low-temperature region and the drive system in the medium-temperature region, thereby reducing the demand for the cold-resistant performance of the drive system, and lowering the cost. Second, the temperature of the storage region is made lower than that of the equipment region through the setting of the temperature isolation device and the evaporator, so that the drive system may be in a relatively high-temperature environment, and it is not necessary to use a drive system with good low-temperature resistance, which reduces the cost. Third, in addition to installing the evaporator in the region of the cabinet body corresponding to the storage region, the evaporator is also installed in the temperature insulation region to ensure a more uniform temperature in the storage region and guarantee the storage effect of the biological sample. Fourth, through the installation of the maintenance port, the drive system may be removed and replaced, which improves the efficiency of the equipment maintenance and meets the repair outside the cabinet, which reduces the loss of air-conditioning caused by the maintenance process and ensures the low-temperature storage of the biological sample. Fifth, through the installation of the positioning groove and the positioning pin, the manipulator mechanism and the drive system may meet the process of loading the cabinet body to achieve fast and accurate positioning. Sixth, the storage region may adapt to a variety of layouts of the sample shelves, with a wide range of applicability. Seventh, the space utilization of the cabinet body is improved from multiple dimensions, which increases the storage capacity of the biological samples. Eighth, the structure is simple and easy to prepare, which enables the realization of large-scale production. Ninth, the shelf layout is optimized, which greatly improves the storage capacity of the samples. Tenth, the structure of the manipulator mechanism is optimized, which makes it easy to pick up and place the material from different directions, and at the same time, it may control the space occupied by the overall activities; through the rotating center offset mechanism, it is convenient to realize the non-round rotation of the rotating rack, which is suitable for the transfer of the material in the narrow channel; During the different turning processes of the rotating rack, adjusting the position of the rotating center point may also ensure the longitudinal guiding column to avoid. Eleventh, by minimizing the gaps between shelves as much as possible, the overall space occupation is controlled. Twelfth, through the combination of camera and light source, it is convenient to monitor the placement of the material in the rotating rack. Thirteenth, through the setting of infrared range finder, it can give feedback on the intelligent monitoring of the material reaching out and reaching into the rotating rack. Fourteenth, by setting the first wiring box and the second wiring box in at least two directions in different positions, the cold volume of the preservation cabinet is more difficult to leak through the first wiring box and the second wiring box, which is conducive to the storage of the biological sample. It should be noted that beneficial effects that may be produced by different embodiments are different, and the beneficial effects that may be produced in different embodiments may be any one or a combination of any of the foregoing, or any other beneficial effect that may be obtained.

Having thus described the basic concepts, it may be rather apparent to those skilled in the art after reading this detailed disclosure that the foregoing detailed disclosure is intended to be presented by way of example only and is not limiting. Although not explicitly stated here, those skilled in the art may make various modifications, improvements, and amendments to the present disclosure. These alterations, improvements, and amendments are intended to be suggested by this disclosure and are within the spirit and scope of the exemplary embodiments of the present disclosure.

## Claims

1. A low-temperature preservation cabinet for a biological sample, comprising: a cabinet body, wherein the cabinet body is provided with a storage region and an equipment region, the storage region is configured to store the biological sample; and
a temperature isolation region is provided between the storage region and the equipment region, wherein a temperature of the storage region is lower than a temperature of the equipment region.

2. The low-temperature preservation cabinet of claim 1, further comprising a sampling mechanism and a refrigeration mechanism;
the storage region being provided with a biological sample rack and a sampling travel channel, wherein the biological sample rack is configured to place the biological sample, and a gap or a temperature isolation device is provided between the storage region and the equipment region;
the sampling mechanism including a manipulator mechanism and a drive system, wherein the drive system is mounted in the equipment region, the drive system is configured to drive the manipulator mechanism to move in the sampling travel channel, and the manipulator mechanism is configured to pick up and place the biological sample; and
the refrigeration mechanism being configured to control the temperature of the storage region to be within a temperature range not higher than -70 °C and to control the temperature of the equipment region to be within a temperature range not lower than -50 °C.

3. The low-temperature preservation cabinet of claim 2, wherein the equipment region is provided above, below, or at a side surface of the storage region.

4. The low-temperature preservation cabinet of claim 2, wherein a gap is provided between the storage region and the equipment region, and the gap takes any value within a range of 80 mm-200 mm.

5. The low-temperature preservation cabinet of claim 2, wherein the equipment region is provided above the storage region, and the temperature isolation device includes a temperature isolation foam.

6. The low-temperature preservation cabinet of claim 2, wherein a temperature isolation device is provided between the storage region and the equipment region, the temperature isolation device is provided with a passageway connecting the storage region and the equipment region; and one end of the manipulator mechanism passes through the passageway and is connected to the drive system.

7. The low-temperature preservation cabinet of claim 2, wherein the cabinet body is provided with a maintenance port, the sampling mechanism includes a mounting frame, the drive system is mounted on the mounting frame, and the mounting frame, the drive system, and the manipulator mechanism are configured to slide and pull out of the maintenance port simultaneously.

8. The low-temperature preservation cabinet of claim 7, wherein support strips are symmetrically provided on opposite side walls in the cabinet body, the support strips extend towards the maintenance port, the mounting frame is a rectangular frame, and two sides of the mounting frame are placed on the support strips.

9. The low-temperature preservation cabinet of claim 7, wherein one end of each of the support strips back away from the maintenance port is provided with a bump perpendicular to the support strip, the bump is provided with a positioning groove, and the mounting frame is provided with a positioning pin corresponding to the positioning groove.

10. The low-temperature preservation cabinet of claim 7, wherein the drive system includes a vertical drive motor, a transverse drive motor, a longitudinal drive motor, and a three-axis transmission mechanism mounted on the mounting frame, the three-axis transmission mechanism is connected with the manipulator mechanism, the vertical drive motor, the transverse drive motor, and the longitudinal drive motor respectively, wherein
the vertical drive motor drives the manipulator mechanism to move along a vertical direction through the three-axis transmission mechanism;
the transverse drive motor drives the manipulator mechanism to move along a horizontal and transverse direction through the three-axis transmission mechanism; and
the longitudinal drive motor drives the manipulator mechanism to move along a horizontal and longitudinal direction through the three-axis transmission mechanism.

11. The low-temperature preservation cabinet of claim 2, wherein the biological sample rack includes a first rack body and a second rack body, the first rack body is symmetrically provided on two opposite inner walls of the cabinet body, the second rack body is provided in the first rack body and parallel to the first rack body, the second rack body is configured as a back-to-back bidirectional rack body.

12. The low-temperature preservation cabinet of claim 2, wherein the manipulator mechanism includes:
a pick-up arm configured to pick up and place the biological sample; and
a steering mechanism, including a rotating rack, a manipulator motor, and an offset guiding mechanism, wherein the pick-up arm is provided on the rotating rack, and the offset guiding mechanism is connected with the rotating rack; and the manipulator motor is connected respectively to the rotating rack and the offset guiding mechanism for driving the rotating rack to rotate and for driving the offset guiding mechanism to move to drive the rotating rack to slide in a center axis direction during rotation.

13. The low-temperature preservation cabinet of claim 1, wherein the cabinet body includes an accommodating cavity, the accommodating cavity includes the storage region, the equipment region, and the temperature isolation region; the storage region is further configured to place a manipulator mechanism, the equipment region is configured to place a driving device, the driving device is configured to drive the manipulator mechanism to pick up and place the biological sample, and the temperature isolation region is provided with a temperature isolation device; and
the low-temperature preservation cabinet further includes an evaporator, the evaporator is provided in a region of the cabinet body corresponding to the storage region, and in the temperature isolation region, so as to cooperate with the temperature isolation device to cause a temperature of the storage region to be lower than a temperature of the equipment region.

14. The low-temperature preservation cabinet of claim 1, wherein the temperature isolation device includes:
a divider member, configured to connect a portion of the cabinet body forming the storage region and a portion of the cabinet body forming the equipment region, to minimize heat conduction between the portion of the cabinet body forming the storage region and the portion of the cabinet body forming the equipment region the portions.

15. The low-temperature preservation cabinet of claim 14, wherein the divider member at least includes:
a first divider portion extending in a first direction;
a second divider portion extending in a second direction different from the first direction; and
a connection portion configured to connect the first divider portion and the second divider portion.

16. The low-temperature preservation cabinet of claim 15, wherein
the connecting portion is provided with one of an insertion projection and an insertion slot, and the first divider portion and the second divider portion are provided with another of the insertion projection and the insertion slot.

17. The low-temperature preservation cabinet of claim 13, further comprising:
at least one mounting box, wherein each of the at least one mounting box is mounted in the temperature isolation region and is provided with at least a portion of the evaporator, a side of the mounting box near the storage region is provided with an opening, and a side of the mounting box close to the equipment region is of a closed structure.

18. The low-temperature preservation cabinet of claim 17, wherein the at least a portion of the evaporator provided within the mounting box is provided on the side of the mounting box close to the storage region, and the temperature isolation device includes:
a temperature isolation member provided on the side of the mounting box close to the equipment region to reduce a cooling capacity generated by the at least a portion of the evaporator within the mounting box to be transferred to the equipment region.

19. The low-temperature preservation cabinet of claim 17, wherein
the at least one mounting box includes a plurality of mounting boxes spaced apart.

20. The low-temperature preservation cabinet of claim 19, further comprising a compressor provided on a first side of the cabinet body and located outside the accommodating cavity, wherein the compressor is configured to form a refrigeration system together with the evaporator; and the plurality of mounting boxes includes:
a first mounting box provided in a region of the temperature isolation region corresponding to the first side;
a second mounting box provided in a region of the temperature isolation region corresponding to a second side opposite the first side; and
the evaporator is configured to be connected to the compressor after first entering the second mounting box and then the first mounting box.

21. The low-temperature preservation cabinet of claim 20, wherein the plurality of mounting boxes further includes:
a third mounting box, wherein the third mounting box is provided between the first mounting box and the second mounting box, and the third mounting box is connected with the first mounting box by a connection member, so as to allow the evaporator to first enter the second mounting box, then enter the first mounting box, then enter the third mounting box through the connection member, then enter the first mounting box through the connection member and then to be connected with the compressor, and the third mounting box is spaced apart from the second mounting box.

22. The low-temperature preservation cabinet of claim 13, wherein
the equipment region is located above the storage region.

23. The low-temperature preservation cabinet of claim 1, wherein
the cabinet body is provided with a maintenance port;
the storage region is provided with a biological sample rack and a sampling travel channel, and the biological sample rack is configured to place the biological sample; and
the equipment region is provided with a mounting frame, and a drive system fixedly connected to the mounting frame, the drive system is connected to a manipulator mechanism and configured to drive the manipulator mechanism to move in the sampling travel channel, the manipulator mechanism is configured to pick up and place the biological sample; and the mounting frame is configured to slide and pull out of the maintenance port.

24. The low-temperature preservation cabinet of claim 23, wherein support strips are symmetrically provided on opposite side walls in the cabinet body, the support strips extend towards the maintenance port, the mounting frame is a rectangular frame, and two sides of the mounting frame are placed on the support strips.

25. The low-temperature preservation cabinet of claim 24, wherein one end of each of the support strips back away from the maintenance port is provided with a bump perpendicular to the support strip, and the bump is provided with a positioning groove, and the mounting frame is provided with a positioning pin corresponding to the positioning groove.

26. The low-temperature preservation cabinet of claim 23, wherein the equipment region is provided above, below, or on a side surface of the storage region.

27. The low-temperature preservation cabinet of claim 23, wherein the drive system includes:
a vertical drive assembly, including a vertical drive motor, a vertical screw, a mounting block, a vertical drive chain, and a first follower wheel; the vertical screw, the vertical drive motor, and the first follower wheel are fixed to the mounting block, respectively, a first gear is coaxially mounted on an output shaft of the vertical drive motor, and the vertical drive chain is meshed and connected with the first gear and the first follower wheel respectively; and the manipulator mechanism is socketed to a rod body of the vertical screw and is fixedly connected with the vertical drive chain.

28. The low-temperature preservation cabinet of claim 27, wherein the drive system includes:
a two-dimensional linear slide mechanism, wherein the two-dimensional linear slide mechanism is connected to the vertical drive assembly to drive the vertical drive assembly to move in a horizontal and transverse direction and a horizontal and longitudinal direction.

29. The low-temperature preservation cabinet of claim 28, wherein the two-dimensional linear slide mechanism includes a transverse drive motor, a transverse screw, a mounting plate, and a transverse rack belt, wherein
the transverse screw and the transverse rack belt are fixed to the mounting plate respectively, the mounting block is socketed to the rod body of the transverse screw, the transverse drive motor is fixed to the mounting plate, the output shaft of the transverse drive motor is coaxially provided with a second gear, and the second gear is engaged with the transverse rack belt.

30. The low-temperature preservation cabinet of claim 29, wherein the two-dimensional linear slide mechanism includes a longitudinal drive motor, a longitudinal drive chain, a second follower wheel, and a guiding strip, wherein
the longitudinal drive motor, the guiding strip, and the second follower wheel are fixed on the mounting frame, a third gear is coaxially provided on an output shaft of the longitudinal drive motor, and the longitudinal drive chain is connected in mesh with the third gear and second follower wheel respectively; and the guiding strip is clipped on the mounting plate, and the mounting plate is connected to the longitudinal drive chain.

31. The low-temperature preservation cabinet of claim 23, wherein the manipulator mechanism includes:
a pick-up arm configured to pick up and place the biological sample;
a steering mechanism, including a rotating rack, a manipulator motor, and an offset guiding mechanism, wherein the pick-up arm is provided on the rotating rack, and the offset guiding mechanism is connected with the rotating rack; and the manipulator motor is connected respectively to the rotating rack and the offset guiding mechanism for driving the rotating rack to rotate and for driving the offset guiding mechanism to move to drive the rotating rack to slide in a center axis direction during rotation.

32. The low-temperature preservation cabinet of claim 1, further comprising a sampling mechanism, wherein the sampling mechanism includes a drive system and a manipulator mechanism for picking up and placing material, and the drive system drives the manipulator mechanism to move;
the cabinet body is fixed with class A shelves and class B shelves;
the class A shelves close to or abut at least one side wall of the cabinet body;
the class B shelves set two by two adjacent to each other or close to each other and back to each other as a class B shelf set;
spacing channels for the manipulator mechanism to move are provided on two sides of a width direction of the class B shelf set and one side of the class A shelves that is away from the side wall of the cabinet body; and
a width of each of the spacing channels is greater than or equal to 0.5 times a width of each of the class A shelves or the class B shelves.

33. The low-temperature preservation cabinet of claim 32, wherein the class A shelves are close to or abut the cabinet body at two side walls or one side wall in a second straight line direction;
a sum of the class A shelves and class B shelves is an integer greater than or equal to 3;
a single class A shelf is designated as one shelf unit and a single class B shelf set as another shelf unit; and
all shelf units are arranged in the second straight line direction with a spacing channel provided between adjacent shelf units, and a count of spacing channels is (a count of class B shelves + 2)/2.

34. The low-temperature preservation cabinet of claim 32, wherein the class A shelves are close to or abut at least three side walls of the cabinet body.

35. The low-temperature preservation cabinet of claim 32, wherein the class B shelves are provided with a clearance channel for the manipulator mechanism to move and yield.

36. The low-temperature preservation cabinet of claim 35, wherein the drive system is the sampling mechanism;
a guiding post clearance is provided on the class B shelves or between the class B shelves and the cabinet body for a longitudinal guiding post of the sampling mechanism to move;
the guiding post clearance communicates with a clearance space, and the clearance space is located at a top of the class B shelves.

37. The low-temperature preservation cabinet of claim 1, further comprising a material lifting device including a pick-up arm for picking up and placing material, wherein
the material lifting device further includes a steering mechanism, wherein the steering mechanism includes a base, a manipulator motor, and a rotating rack, the manipulator motor is mounted on the base, and the manipulator motor is configured to drive the rotating rack to rotate;
the rotating rack is provided with the pick-up arm;
the material lifting device further includes a rotating center offset mechanism, the rotation center offset mechanism is configured to drive the rotating center axis of the rotating rack to translate along a first linear direction when the rotating rack is moving, and the manipulator motor is configured to drive the rotating center offset mechanism to move; and
the center axis direction of the rotating rack is perpendicular to the first linear direction.

38. The low-temperature preservation cabinet of claim 37, wherein the pick-up arm includes a sliding member for picking up and placing material, and the sliding member is slidably connected with the rotating rack through a guide rail; and
the pick-up arm further includes a pick-up drive mechanism to drive the sliding member to move linearly along the guide rail.

39. The low-temperature preservation cabinet of claim 37, further comprising a mounting base, wherein the mounting base is configured to support the steering mechanism; a side of the mounting base is provided with a guiding hole for slidingly connecting a guiding post; and
when the rotating center axis translates in the first linear direction, the rotating center axis moves from a side close to the guiding hole to a side away from the guiding hole or moves from the side away from the guiding hole to the side close to the guiding hole.

40. The low-temperature preservation cabinet of claim 37, further comprising a mounting base, wherein the mounting base is configured to support the steering mechanism; a side of the mounting base is provided with a guiding hole for slidingly connecting a guiding post; and
the manipulator motor is located on either side of the rotating rack in the center axis direction of the guiding hole.

41. The low-temperature preservation cabinet of claim 40, wherein the rotating center offset mechanism includes a guiding assembly configured to guide the base to be slidably connected with the mounting base in the first linear direction.

42. The low-temperature preservation cabinet of claim 41, wherein the rotating center offset mechanism further includes a connecting rod, one end of the connecting rod is hinged to the base, and another end of the connecting rod is hinged to the rotating rack.

43. The low-temperature preservation cabinet of claim 37, wherein the rotating center offset mechanism includes a guiding assembly configured to guide the rotating center axis of the rotating rack to slide in the first linear direction.

44. The low-temperature preservation cabinet of claim 43, wherein the guiding assembly includes a slide rail and a slide block;
a center of the slide block is rotationally connected to the rotating rack by a rotating shaft;
a power output end of the manipulator motor is connected to one end of an oscillating arm through a gear drive mechanism, another end of the oscillating arm is movably connected to a guiding rod, and the guiding rod is connected to the rotating rack; and
the base and the slide rail are mounted on the mounting base, the mounting base is provided with a guiding groove for guiding the guiding rod to move, and the guiding groove is U-shaped.

45. The low-temperature preservation cabinet of claim 37, wherein the rotating rack is provided with a camera device and a light source adjacent to the camera device.

46. The low-temperature preservation cabinet of claim 37, wherein the cabinet body is provided with shelves, at least one side of the shelves in a width direction is provided with a passageway for the material lifting device to move, and a length direction of the passageway is parallel to the first linear direction.

47. The low-temperature preservation cabinet of claim 39, wherein shelves are provided within the cabinet, and a passageway is provided between two adjacent shelves for the material lifting device to move, and a length direction of the passageway is parallel to the first linear direction;
the passageway is directly opposite a shipping port, and the shipping port is located on a side of the mounting base away from the guiding hole;
an orientation of the pick-up arm is adjusted during rotation of the rotating rack, and the pick-up arm is oriented in sequence toward one of the two adjacent shelves, the shipping port, and another one of the two adjacent shelves;
the rotating center axis of the rotating rack is located on the side close to the guiding hole when the pick-up arm is oriented toward either of the two adjacent shelves; and
the rotating center axis of the rotating rack is located on the side away from the guiding hole when the pick-up arm facing the shipping port.

48. The low-temperature preservation cabinet of claim 1, wherein the cabinet body includes an outer shell defining a shell cavity and an inner liner provided in the shell cavity, the inner liner defines an accommodating cavity, the outer shell is provided with a first wiring port, the inner liner is provided with a second wiring port, and the first wiring port and the second wiring port have different positions in at least two directions; and
the accommodating cavity is provided with a drive system, the drive system is connected to one end of a cable, another end of the cable extends out of the shell cavity after passing through the second wiring port and the first wiring port, and the cable is configured to realize power supply and/or control of the drive system.

49. The low-temperature preservation cabinet of claim 48, wherein
a mounting mechanism is fixed to an outer wall of the outer shell, the mounting mechanism is provided with a control device, and the control device is connected to another end of the cable.

50. The low-temperature preservation cabinet of claim 48, wherein
the first wiring port is configured to place a first wiring box, the second wiring port is configured to place a second wiring box, the another end of the cable passes through the second wiring port via the second wiring box, and the another end of the cable passes through the first wiring port via the first wiring box.
